# EUROPEAN PATENT APPLICATION

(11) **EP 3 026 426 A1**
(43) Date of publication of application: **01.06.2016**
(21) Application number: 14003985.0
(22) Date of filing: 26.11.2014
(51) Int. Cl.: G01N 21/552, G01N 21/85, G01N 21/43

(54) **A measuring probe, an apparatus and a method for label free attenuated reflection infrared spectroscopy**

(71) Applicant: Universität Stuttgart, 70174 Stuttgart (DE)
(72) Inventor: Körner, Klaus, 70563 Stuttgart (DE); Claus, Daniel, 70195 Stuttgart (DE); Herkommer, Alois, 73431 Aalen (DE); Osten, Wolfgang, 70569 Stuttgart (DE)
(74) Representative: Müller-Boré & Partner Patentanwälte PartG mbB

(57) **Abstract**

The present invention relates to a measuring probe for infrared spectroscopy, said measuring probe having an elongated form with a first end for coupling and decoupling infrared light into and out of the measuring probe and a second end. The measuring probe comprises an ATR prism arranged at the second end of the measuring probe. The ATR prism has at least one first surface having at least one measuring portion configured to be brought in optical contact with a measured object, at least one second surface having at least one reflective portion, and a cutting portion (53; 54) for cutting through the measured object, said cutting portion having a cutting tip or cutting blade with a cutting angle of equal to or less than 60°. The ATR prism is configured so that at least a portion of the infrared light entering the measuring probe undergoes an attenuated total reflection at the least one measuring portion of the first surface, at least a portion of the totally reflected light is reflected back towards the first surface by the at least one reflective portion of the second surface, and at least a portion of the light reflected back by the at least one reflective portion of the second surface undergoes an attenuated total reflection at the at least one measuring portion of the first surface and is decoupled from the ATR prism.

The invention relates further to an apparatus for infrared spectroscopy comprising the measuring probe and a method for infrared spectroscopy.

## Description

A standard approach ("gold standard") in cancer diagnosis is a hystopathologic examination of a tissue sample in question, which is usually done by an experienced hystopathologist in a specialized laboratory. In order to carry out the examination, tissue samples are extracted from the patient by means of a biopsy. A disadvantage of this approach is that it is slow, labor-intensive and causes a considerable discomfort to the patient.

As an alternative to the hystopathologic examination, various infrared spectroscopy methods and in particular infrared Fourier spectroscopy methods for tissue analysis have been proposed. However, the majority of these methods require the extraction of a tissue sample via biopsy, usually under the application of an anesthetic. The extracted tissue sample is subsequently cut in thin slices by a microtom, the slices are further thinned and optionally dried and/or chemically treated and finally subjected to an infrared spectroscopic examination in a transmission or transflection mode. Exemplary infrared spectroscopy methods for tissue analysis are disclosed in the publication Hughes, Caryn [Thesis] Development of Fourier Transform Infrared Spectroscopy for Drug Response Analysis, University of Manchester, 2011.

WO 2009/137122 discloses an ATR prism (Attenuated Total Reflection prism) based spectroscopic method, which utilizes spectral measurements around the critical total reflection angle in infrared range by means of a Fourier transform spectroscopy. A disadvantage of this method is that it is relatively slow, as it requires a high precision angular scan of the ATR prism and the detection and processing of many spectral images obtained at different angular positions.

US 6,507,395 B1 discloses an ATR based infrared spectroscopy apparatus. The proposed apparatus is, however, bulky and cannot be easily miniaturized.

US 7,697,976 discloses a Fourier transform spectrometer in combination with a miniaturized ATR based measuring probe in a catheter or hollow fiber form that may be used for cancer diagnosis. When performing spectroscopic measurements the ATR prism is placed on the skin surface. Due to the relatively obtuse angle of the ATR prism and the material (ZnSe) from which it is made, the ATR prism is not suitable for carrying out measurements in the depth of an observed object (e.g. a living organ).

US 8,452,356 B2 discloses a fiber-based ATR approach to infrared spectroscopy using two fibers for analysis of physiological fluids. Illumination light is coupled into one of the fibers and reflected by a retro-reflector in the other fiber. Due to the volume of the retro-reflector, the measuring site cannot be precisely determined.

US 7,952,719 discloses a catheter employing a Raman spectroscopy coupled with OCT to perform a scanning in the depth of the measured object (for example human tissue) and obtain morphological information.

The publication Y. Matsuura "Hollow-fiber-based flexible probe for remote measurement of infrared attenuated total reflection", published in Applied Optics, Vol. 48, No. 28, Oct. 2009, S. 5396 - 5400 describes a hollow fiber with an ATR prism for carrying out spectroscopic measurements of human tissue. To perform the measurements the ATR prism is placed on the surface of the measured organ.

US 2011/0306956 A1 discloses a method and a catheter for selective, spatially addressed human tissue treatment by an absorption of laser light. The catheter is, however, not suitable to use an ATR prism.

DE 69233647 discloses an apparatus for tumor hypothermic treatment with a hollow needle having an outer diameter up to 3 mm. The treatment is carried out without any sensing during the treatment, which reduces the accuracy and the success rate of the treatment.

One object of the invention is to provide an improved, minimally invasive infrared spectrometer which is suitable for carrying out measurements of biological and technical objects, even at greater depths in the inside of the measured object. In particular, an object of the invention is to provide a minimally invasive infrared spectrometer for carrying out in-vivo measurements on human or animal tissue, preferably during a surgical intervention. Another object of the invention is to improve the accuracy and reliability of the infrared spectroscopy measurements and to reduce the measuring time. Further objects may to provide a multi-sensor apparatus, capable of integrating a plurality of optical measurement approaches and/or an apparatus capable of carrying out both optical measurements and treatments of the observed object (for example laser treatment for cancer therapy).

According to an aspect the above objects are solved by the provision of a measuring probe, an apparatus for infrared spectroscopy and a method for infrared spectroscopy, as defined in the independent claims, respectively. Preferred embodiments are defined in the dependent claims.

Applications of the proposed measuring probe, apparatus and method for infrared spectroscopy include but are not limited to measurements on biological objects and materials, for example to obtain biospectral information of human or animal tissue samples, cells, etc. for cancer diagnostic, diagnostic of complex metabolic processes, time resolved reaction kinetic analysis, etc. The proposed measuring probe, apparatus and method for infrared spectroscopy are particularly suitable for minimally invasive in-vivo label-free optical measurements and optionally treatment in the depths of the prostate, lungs, stomach, breasts and/or other human or animal organs. The measurements may be carried out during a surgical intervention such as biopsy or surgery, during drug administration, tumor screening, etc.. Further applications include measurements of the properties of cells, tissue, living organisms and/or other biologic materials for basic biological, medical and veterinary research, drug research, forensic. Applications may also include measurements of the properties of non-biological materials, for example for material testing, quality control, etc..

Preferably, the proposed measuring probe, apparatus and method for infrared spectroscopy enables reliable and traceable measurements at different spatial points of the measured object (including points in the depth of the measured object) with a lateral resolution in the sub-millimeter range, for example from about 2 mm to about 0.5 mm. If the measurements are repeated, the lateral resolution may be improved, in particular in the middle infrared range (MIR), for example for wavenumbers about 1000 cm⁻¹ (corresponding to about 10 µm wavelength). Such spatial resolution is considered to be very good for diagnostic purposes during surgery and sufficient for many other applications. For research applications, in particular under controlled laboratory conditions, the employment of brilliant light sources and diffraction limited optical arrangements, it is possible to obtain a spatial resolution in the upper two-digit micrometer range.

Further, preferably, the proposed measuring probe, apparatus and method for infrared spectroscopy may be configured for label-free multi-sensor optical measurements. Thus, it is possible to combine infrared spectroscopic data with data obtained on the basis of further optical measurements, for example morphologic data obtained by an optical coherence tomography and/or microscopy, data obtained on the basis of Raman spectroscopy, etc.

According to an aspect there is provided a measuring probe for an infrared spectroscopy. The measuring probe has an elongated form with a first end for coupling and decoupling infrared light emitted from the light source into and out of the measuring probe and a second end (front end of the measuring probe that comes first in contact with the measured object). The measuring probe comprises an ATR prism arranged at the second end of the measuring probe, the ATR prism having
at least one first surface having at least one measuring portion configured to be brought in optical contact with a measured object,
at least one second surface having at least one reflective portion, and
a cutting portion for cutting through the measured object, said cutting portion having a cutting tip or cutting blade with a cutting angle of equal to or less than 60°, preferably equal to or less than 45°, further preferably equal or less than 30°, still further preferably equal or less than 25°.

The ATR prism is configured so that
at least a portion of the infrared light entering the measuring probe undergoes an attenuated total reflection at the least one measuring portion of the first surface,
at least a portion of the totally reflected light is reflected back towards the first surface by the at least one reflective portion of the second surface, and
at least a portion of the light reflected back by the at least one reflective portion of the second surface undergoes an attenuated total reflection at the at least one measuring portion of the first surface and is decoupled from the ATR prism.

According to a further aspect of the invention there is provided an apparatus for infrared spectroscopy (infrared spectrometer), comprising a light source, a detector and a measuring probe according to any of the aspects of the invention.

The measuring probe and the respective apparatus for infrared spectroscopy comprising the measuring probe employ a novel ATR prism (Attenuated Total Reflection prism) as an optical element for coupling the illumination light into the measured object by means of an attenuated total reflection. The term "ATR prism" is used to cover any optical element that is configured to couple and decouple infrared light into the measured object by means of an attenuated total reflection having a prism, pyramid, plate, conical, or other suitable form.

The proposed ATR prism has a surface having a reflective portion thereon configured such that at least a part of the incoming light (i.e. infrared light entering through the first end of the measuring probe and passing through an optical coupling surface of the ATR prism) undergoes twice an attenuated total reflection at the measuring portion of the first surface which is in optical contact with the measured object to form an output (exiting) light, which is subsequently detected by a detector. The provision of a reflective portion for back reflection towards the measuring portion in optical contact with the object, so that the reflected light undergoes an attenuated total reflection for a second time, allows for a considerable enhancement of the measured signal. This improves the sensitivity of the measurements and reduces the measuring time. In the following the first surface, which has a measuring portion in optical contact with the measured object, where measurements are carried out by means of attenuated total reflection will be referred to as a measuring surface. The second surface, which has a reflective portion to guide the light back to the measuring portion, will be referred to as a reflective surface.

In case the ATR prism is monolithically built (i.e. built as a single optical element with fixed surfaces and angles), the provision of a reflective portion has the additional technical effect of the invariance of the average of the magnitudes of the two incident angles alpha_e1 and alpha_e2. The angle alpha_e1 is the angle of incidence of the incoming light at the measuring portion of the measuring surface, i.e. the angle between the normal to the measuring portion and the main ray of the beam of light incident on the measuring portion prior to reflection by the reflective portion of the reflective surface. The angle alpha_e2 is the angle of incidence of the light beam reflected by the reflective portion of the reflective surface at the measuring portion of the measuring surface, i.e. the angle between the normal to the measuring portion and the main ray of the beam of light reflected by the reflective portion of the reflective surface and incident on the measuring portion of the measuring surface.

In other words, the average value alpha_av, alpha_av = (alpha_e1 + alpha_e2)/2, is invariant with respect to changes of each individual incident angle alpha_e1 + alpha_e2.

In particular, in case of a monolithic prism with an angle alpha_p between the measuring surface and the reflective surface and more precisely between the measuring portion of the measuring surface and the reflective portion of the reflective surface, the condition alpha_p = alpha_av is fulfilled. Accordingly, the average value alpha_av is independent of each individual angle of incidence alpha_e1 or alpha_e2. Thus, the ATR-prism having a reflective surface provided with a reflective portion exhibits invariance of the average value alpha_av with respect to the angles of incidence alpha_e1 + alpha_e2. In the following the angle alpha_p will be referred to as prism angle.

Generally, the average value alpha_av may be preferably equal to or greater (or, in some very special cases, smaller) than the critical total reflection angle, wherein the difference may be equal or less than 12°, equal to or lower than 10°, further preferably equal to or lower than 3°. The critical total reflection angle depends on refractive index n_m of the object or medium which is to be measured and the refractive index n_p of the material forming the ATR prism (critical total reflection angle = arcsin (n_m/n_p)). The critical angle may be computed for each particular application of the infrared spectrometer. For infrared, the refractive index of the material of the ATR prism is typically in the range of 2.38 to 4.1 at the respective wavelength.

The proposed approach of employing an ATR prism with a reflection portion, configured such that the light undergoes a double attenuated total reflection at the measuring portion increases the robustness of the spectroscopic measurements, since the average value alpha_av of the two incident angles alpha_e1 and alpha_e2 for the first and the second total reflection is a single variable invariant with respect to the changes of each individual incident angle. If for example, the first incident angle alpha_e 1 for the first total reflection is reduced due to a tilt of the ATR prism or the axis of the incident light beam (for example after a change of the light source), the second incident angle alpha_e2 is automatically increased. Accordingly, the variation of the first incident angle alpha_e1 may be compensated to a certain extent. This allows to guarantee and increase the stability (including the long-term stability) and robustness of the spectroscopic measurement.

Further, the variation of the incident angle alpha_e1 may be easily detected via a detection of the angle beta, which is the angle between the main ray of the light beam incident on the measuring portion of the measuring surface and the main ray of the light after double total reflection at the measuring portion of the measuring surface. Based on the measurement, a manual or automatic correction of the incident angle may be undertaken to compensate for any variation of the incident angle. The angle beta may range for example from 0° to 24°, preferably from 0° to 5°. Accordingly, the angle beta/2 may range from 0° to 12°, preferably from 0° to 2.5°. Preferably the angle beta is corrected manually or automatically, so that it is set at a small value, preferably a value under 5°, further preferably under 2°. This value enables a good coupling of the incident light beam entering the ATR prism (incoming light beam) and decoupling of the light beam that has undergone a double attenuated total reflection.

Preferably, the ATR prism angle alpha_p is adjusted to a specific value depending on the optical properties (e.g. refractive index and absorption constant) of expected measured object, for example expected cancer tissue. In other words, the ATR prism may be tuned for a detection of specific objects or materials, such as specific types of cancer tissue. This increases the sensitivity of the detection of these objects or materials.

In an example, a monolithic ATR prism, in particular a diamond ATR prism, can be manufactured with a high accuracy, so that the prism angle alpha_p reaches a predetermined value with a tolerance of less than 1/10^{th} angular degrees. Thus, since the prism angle alpha_p is fixed for a given ATR prism, the spectroscopic measurements can be carried out under very stable conditions, which increases the reliability and repeatability of the measurement data, even in case of unstable environment, such as for example during a surgery.

The measuring probe may have an elongated form with a longitudinal axis that may be parallel or nearly parallel to the direction of insertion of the measuring probe. In an example, the longitudinal axis of the measuring probe may be at an angle to the insertion direction. This may facilitate the insertion of the measuring probe into the measured object. The measuring probe may have a hollow needle form, a catheter form, a hollow fiber form or any other suitable form, which facilitates the insertion of the measuring probe into the measured object (for example a living organ). For example, the measuring probe may have an elongated, hollow cylindrical or conical form that may narrow down towards the second end of the ATR prism where the ATR prism is arranged.

The ATR prism may be arranged in an upright (vertical arrangement). Preferably, the angle between the measuring surface and the longitudinal axis of the measuring probe may be smaller than 60°, preferably smaller than 45°, more preferably smaller than 25°. Similarly, the angle between the reflective surface and the longitudinal axis of the measuring probe may be smaller than 60°, preferably smaller than 45°, more preferably smaller than 25°.

The above arrangement of the ATR prism in combination with the provision of a cutting portion at an end (front end) of the measuring probe allows realizing an ATR prism having both an optical and a cutting function.

In an example, the cutting portion may be formed by an intersection of the first surface and the second surface of the ATR prism. The first surface (measuring surface) and the second surface (reflective surface) may intersect at a common point (e.g. an apex of a pyramid ATR prism) or along a common line of intersection, thereby forming a cutting blade for cutting through the measured object. In another example, the cutting portion may be an additional element that may be in fixed mechanical arrangement (fixed mechanical contact) with respect to the remaining parts of the ATR prism. In both cases, the cutting blade is at the front end of the measuring probe that is first inserted into the measured object and cuts through it.

In the first example (cutting portion formed by intersection of the measuring and reflective surfaces), the cutting angle of the cutting blade is equal to the prism angle alpha_p, which is selected such that there is a double attenuated total reflection on the measuring portion and which depends on the refractive index of the ATR prism material and the refractive index of the measured object. Depending on the material of the prism, this may prevent the realization of very sharp cutting blades. One advantage of this arrangement may be that at least one cutting surface or a part thereof may serve as a measuring portion, thereby allowing spectroscopic measurements also at object positions close to or in immediate vicinity of the cutting portion. Thus, the zone around the front end of the measuring probe not accessible to measuring light may be reduced or altogether eliminated, thereby allowing optical measurements in the depth of the measured object.

In the latter example (cutting portion formed as an additional element), the cutting portion may be made of a non-transparent hart material, which is suitable for cutting through the measured object (such as stainless steel). Preferably, the cutting portion is made of an optically transparent material, such as for example diamond, crystalline silicon, crystalline germanium, etc. Further preferably the ATR prism with the cutting portion has a monolithic structure, i.e. the cutting portion has a fixed mechanical, and in case of transparent cutting portion, optical contact with the remaining part of the ATR prism. The cutting surfaces of the cutting portion may be in flush with the first and the second surfaces of the ATR prism, respectively (i.e. with the measuring and the reflective surface of the ATR prism, respectively).

One advantage of an ATR prism comprising a cutting portion which is not formed by an intersection of the measuring an the reflective surface (i.e. a cutting portion formed as an element different in its construction than the other, optically active part of the ATR prism) is that the cutting angle of the cutting portion may be more freely set than the prism angle (which depends on the critical total reflection angle). In particular, the cutting angle (i.e. the angle between the cutting surfaces constituting the cutting portion) may be considerably lower than the prism angle (i.e. the angel between the measuring and the reflective surfaces). Thus, the cutting angle may be preferably ≤30°, more preferably ≤25°. This facilitates the cutting through the measured object and reduces the damages and injuries caused by the insertion of the probe in the measured object. However, in this case parts of the measured objects around the cutting portion may not be accessible to the measuring light.

In both of the above described examples, the ATR has a double function: an optical function and a cutting function. An advantage of an ATR prism having a double function is that the optical measurement may be made in-situ, immediately after the cutting of the measured object. When cutting through soft tissues and other similar materials, as a rule a thin fluid film or layer is formed on the measuring surface of the ATR prism while the ATR prism cuts through the soft tissue. Due to this thin fluid film or layer, there is a good optical coupling between the cut part of the tissue that is to be measured and the measuring surface. Accordingly, it is not necessary to apply additional high pressure, in order to assure good optical contact. This is a considerable advantage over a horizontal ATR approach, which generally requires the application of high pressure in order to ensure good optical contact between the horizontal measuring surface and the measured object, in particular in case the examined soft tissue has been previously cut by a microtome and the drying process of the cut tissue has already started.

The ATR prism may have a prism, a pyramid or a conical form. In some examples, the ATR prism may be made in a plate-like or an optical fiber form. Preferably, the ATR prism and the measuring probe exhibit a diffraction limited optical design. For example the ATR prism may be miniaturized and may have cross-sectional dimensions of about 1 mm x 1 mm, preferably 0.5 mm x 0.5 mm. The lateral extension of the measuring probe (i.e. the cross section of the measuring probe) may be about 2 mm x 2 mm, preferably about 1 mm x 1 mm, most preferably around 0.8 mm x 0.8 mm. This reduces the damages caused to the measured object and enables spatial measurements at relatively high spatial resolution.

Generally, the ATR prism may be configured to work at or around the critical total reflection angle. The difference between the critical total reflection angle and the angle of reflection for which the ATR prism is configured may be up to several angular degrees (≈ ±10°). For example the difference between the angle alpha_p between the first surface and the second surface of the ATR prism and the critical total reflection angle may be equal or lower than ±12°, preferably ≤±10°, further preferably ≤±3°, still further preferably ≤±1°. Thus for example, reflection angles under the critical total reflection angle (such as for example reflection angles of up to 3° under the critical total reflection angle) may produce valuable measuring data, in particular in combination with a comparison to reference data of the measured object, which are obtained in advance (such as reference data of human tissues having known properties, such as known classification into malign or non-malign). Reflection angles above the critical total reflection angle may assure that even if there is angular spread of the incoming beam (for example due to dispersion and/or imperfect collimation), the attenuated total reflection is assured for all portions of the incoming light beam, including the outermost rays.

The optically active part of the ATR prism and optionally the whole ATR prism (including the cutting portion) may be made of any suitable optical material that has a sufficient transparency in the relevant infrared spectral range. For example the ATR prism may be made of diamond, zinc selenite (ZnSe), crystalline germanium (Ge), crystalline silicon (e.g. produced by a floating zone process), etc..

In an example, the ATR prism with the cutting portion is made of diamond. Diamond is optically transparent for a broad spectral range, has a high refractive index, in particularly also in infrared range, and exhibits an excellent durability, chemical inertness and biocompatibility. Due to its high hardness, it is particularly suitable for realizing an ATR prism having a double function: an optical and a cutting function for cutting through the measured object. Further, it is possible to manufacture very small ATR prisms with a high accuracy. This facilitates the manufacturing of very thin, minimally invasive measuring probes.

Crystalline silicon (Si) also exhibits high hardness and good biocompatibility. An additional advantage is that it has a high refractive index (n = 3.4 in infrared), thereby enabling the construction of ATR prisms with very sharp cutting blades having cutting angles ≤30°. Further, due to the high refractive index, a silicon surface behaves almost like a perfect mirror surface when the angle of incidence is equal to or greater than 60° (total reflection without attenuation or non-attenuated total reflection). This effect may be advantageously used to guide an infrared light coupled to the ATR prism to the measuring portion of the measuring surface.

Crystalline germanium (Ge) has also a very high refractive index in infrared (n = 4 for mid infrared range). This allows constructing ATR prisms with very sharp cutting blades having cutting angles ≤25°. Further, as in case of crystalline silicon, it allows to advantageously use the mirror like behavior at high incident angles (for example higher than 50°), to guide the light coupled to the ATR prism to the measuring portion of the measuring surface.

For example, the first and/or the second surface of the ATR prism may comprise at least one reflective portion, configured to reflect incident light by means of non-attenuated total reflection towards the second surface. At this portion, there is substantially no interaction with the measured object, so that no spectroscopic measurement is carried out. However, it is possible to easily realize an optical guide like structure to guide the infrared light to the measuring portion without the application of a reflective layer, thereby reducing the costs of the ATR prism and simplifying the production.

Of course, the first and/or the second surface may comprise at least one reflective region provided with a reflective layer. The reflective layer may be for example a metal layer, such as an aluminum layer. The reflective layer may have a multilayer structure, preferably with a hard coating layer as an outermost layer.

In an example, the first surface of the ATR prism may comprise a plurality of measuring portions, each of them configured to be brought into optical contact with the measured object. This improves the detected measurement signal and enables reducing the measurement time. However, the lateral spatial resolution may be reduced, since the measurement signal is collected from different spatially distant sites.

The reflective surface of the ATR prism may be fully or partially covered with a reflective layer which forms a reflective portion. In an example, the reflective surface has a least one reflective portion and an optically transparent portion (non-reflective portion). This may facilitate performing further optical measurements (for example Raman spectroscopy, optical coherence tomography microscopy, etc.) as it will be explained in more detail further on.

After undergoing an attenuated total reflection at the measuring portion, the light may be reflected once at the reflective portion. However, the overall number of the reflections at the reflective surface may be more than one. Preferably, the number of reflections (including the reflection at the reflective portion and the attenuated total reflections at the measuring portion) is uneven (i.e. 2n+1, with n=1, 2, 3, ...). In this case, it is possible to assure that the average angle alpha_av exhibits invariance with respect to each individual incident angle, as described above for the case of one reflection.

In an example, the ATR prism may comprise a plurality of surfaces, each having at least one measuring portion configured to be brought in optical contact with a measured object. The apparatus for infrared spectroscopy may also comprise a plurality of surfaces, each having at least one reflective portion. In an example, the reflective portions may be provided on each of the surfaces comprising the at least one measuring portion. The plurality of surfaces comprising both measuring and reflective portions may be arranged such that the infrared light undergoes a double attenuated total reflection at each measuring portion of each surface.

For example, the ATR prism may exhibit a pyramid form and the pyramid faces may have at least one measuring portion and at least one reflective portion, arranged such that an attenuated total reflection occurs twice at each measuring portion provided on each of the pyramid faces. Thus, it is possible to realize a very compact multi-channel ATR prism that is capable of simultaneously measuring the spectroscopic properties at at least two different sites of the measured object. At the same time the pyramid faces meeting at the apex of the pyramid may serve as cutting surfaces for cutting through the measured object. Further, it may be possible to obtain a full, 360° measurement at a particular measuring site with a minimum amount and number of rotation of the ATR prism or even eliminating rotation of the ATR prism at all.

In some examples, the number of reflections at each reflective surface may be one. The number of reflections at the reflective surfaces may be more than one, for example three, five, etc.

The infrared spectroscopic measurement may be combined with further optical measurements, all of them using advantageously the same ATR prism as an optical coupling element. To this end the ATR prism (a multi-channel ATR prism) may comprise a plurality of optical coupling surfaces. The ATR prism may comprise a first optical coupling surface for coupling light for the infrared spectroscopic measurement into the ATR prism and optionally decoupling the light after an interaction with the object out of the ATR prism. The ATR prism may further comprise one additional, second optical coupling surface for coupling light for further optical measurements into the ATR prism (and optionally decoupling the light for further optical measurements after interaction with the measured object out of the ATR prism). The further optical measurement may include but is not limited to any of Raman spectroscopy, optical coherence tomography, swept-source spectral-domain optical coherence tomography, microscopic observation (preferably in ultraviolet, visible or near infrared light).

For example, the infrared spectroscopic measurement may be combined with Raman spectroscopy measurement. The Raman spectroscopy may be a spontaneous Raman spectroscopy. Further, the Raman spectroscopy may be a non-linear Raman spectroscopy, such as Coherent Anti-Stokes Raman Scattering (CARS) or stimulated Raman Scattering Spectroscopy (SRS). These coherent techniques offer advantages over spontaneous Raman spectroscopy such as a much higher sensitivity to concentration partly due to the enhanced scattering cross sections. Preferably, the ATR prism is configured such that the Raman excitation light emitted by a suitable light source (for example light source emitting light in the near infrared (NIR), visible (VIS) or ultraviolet (UV) range) passes through the second coupling surface and is directed to the measuring surface of the measuring probe. The scattered Raman light may exit the ATR prism through the second coupling surface or through a respective exit surface for the Raman scattered light and be directed to a suitable detection path for the Raman spectroscopy.

Similarly, it is possible to combine the infrared spectroscopic measurement with other measurements, such as optical coherence tomography (in particular swept-source spectral-domain optical coherence tomography), microscopic observation (preferably in ultraviolet, visible or near infrared light) or any other optical measurements. The ATR prism may comprise more than two optical coupling surfaces for the different types of optical measurements.

The optical coupling surfaces may be plane or curved surfaces (for example convex or concave curved spherical or aspherical surface). Preferably, the measuring probe and the ATR prism are configured such that the light for further optical measurement is incident normally or near normally on the measuring portion of the measuring surface. For example, the at least one optical coupling surface for coupling light for further optical measurement may be arranged such that this surface or a tangential plane to this surface is normal or nearly normal with respect to the measuring surface. Nearly normal incidence means incidence at an angle deviating from the normal (perpendicular) incidence by no more than ±15°, preferably by no more than ±12°. Due to the normal or near normal incidence of the light for further optical measurement to the measuring portion of the measuring surface, this light does not experience a total reflection, so that the photons returned from the measured object may pass through the measuring surface and be directed towards a suitable detector (i.e. directed in a suitable detection path for a second optical measurement and optionally at least one further optical measurement).

The apparatus for infrared spectroscopy employing the multi-channel measuring probe described above may comprise additional light sources (such as additional light sources for Raman excitation light, optical coherence tomography light and/or microscopic observation light), detectors and additional optical forming additional illumination and detection paths. Thus, it is possible to combine at least two, three or more different optical sensors in the same optical arrangement and to carry out further optical measurements, preferably simultaneously with the infrared spectroscopic measurements. The detected data of the different optical measurements may be combined and used to determine the properties of the measured object. For example spectral data may be combined with morphologic data obtained for example by optical coherence tomography and/or microscopy. This may for example improve considerably the identification of unknown objects, for example the classification of an observed tissue as healthy tissue, cancer tissue in different grades, etc.

The apparatus for infrared spectroscopy may comprise a kit or a magazine of measuring probes, wherein preferably each of the probes exhibits an ATR prism with different prism angle (i.e. different angle between the measuring and the reflective surface). The different probes may be easily exchanged when the need arises. In an embodiment, due to the invariance of the average value alpha_av, the alignment of the optical system after a change of the measuring probe is relatively simple and may be carried out automatically.

The remaining components of the apparatus for infrared spectroscopy employing the measuring probe, such as a light source, a detector, spectrometer, etc., may be arranged in a known manner. Preferably the apparatus for infrared spectroscopy is a Fourier transform infrared spectrometer.

For example the apparatus for infrared spectroscopy may employ different light sources, such as broadband light sources emitting light with a substantially continuous spectrum or light sources emitting monochromatic or quasi-monochromatic light with a variable (i.e. scannable) wavelength within a broad spectral range (e.g. so called swept sources). The emitted monochromatic or quasi-monochromatic light may be frequency and/or amplitude modulated. The broadband light source may be combined with a monochromator, a spectrometer or an interferometer, as in known in the art.

The light source may be for example a brilliant light source, such as a synchrotron radiator (emitting preferably in mid-infrared), a broadband quantum cascade laser or a laser battery. Through the use of such brilliant light sources, it is possible to achieve a diffraction limited optical design and miniaturize the measuring probe. Further, the overall measuring time may be reduced. This facilitates the application of the apparatus for infrared spectroscopy for in-vivo tissue analysis, for example during a surgical intervention.

If quantum cascade lasers or other laser sources are used, it is preferable to reduce the time coherence of the illumination light. This reduces or prevents the occurrence of parasitic interferences causing errors in the ATR spectroscopic measurements.

The spectrometer may be any conventional spectrometer, for example a conventional infrared Fourier transform spectrometer. The interferometer may be for example a Michelson-interferometer, a rotatable retro-reflective mirrors based interferometer or any other suitable interferometer. Preferably, the apparatus for infrared spectroscopy is a Fourier transformation infrared spectrometer, thereby realizing an **A**ttenuated **T**otal **R**eflectance **F**ourier **T**ransform Infrared **S**pectroscopy/**S**pectrometer (ATR-FTIR). For example, the ATR-FTIR may employ a broadband quantum cascade laser having an overall power of about 0.4 W and emitting light in the middle infrared spectral range of about 5 µm to about 11 µm (about 2000 cm⁻¹ to about 909 cm⁻¹). The resolution of the ATR-FTIR may be about 4 cm⁻¹ at least, preferably 2 cm⁻¹, more preferably 1 cm⁻¹. This enables the realization of a multi-channel ATR prism based infrared spectroscopic system for a label-free, relatively fast in-vivo diagnostic.

The detector may be an integral detector, for example a spatially and/or spectrally integrally detecting detector. The detector may be a one or two-dimensional detector array with a plurality of detector elements. Suitable detectors for light in the infrared spectral range are for example mercury-cadmium telluride (MCT) detectors. Suitable detectors for light in the visible range are for example CCD cameras.

Further, the apparatus for infrared spectroscopy may comprise a data analysis component configured to process the obtained infrared spectroscopic data and subject them to further analysis. The analysis may include a Fourier transform spectrometric analysis or any other suitable analysis. Further, the analysis may comprise a Principal Component Analysis or other suitable statistical analysis to obtain characteristic features from the obtained spectral data, which may be then used to identify or classify an unknown measured object.

According to a further aspect, there is provided a method for infrared spectroscopy by using a measuring probe and/or an apparatus for infrared spectroscopy according to an aspect of the invention.

The method comprises
bringing the at least one measuring portion of the first surface of the ATR prism in optical contact with the measured object;
illuminating the at least one measuring portion with incident infrared light, so that at least a portion of the incident light undergoes an attenuated total reflection at the at least one measuring portion,
reflecting, by the at least one reflective portion of the second surface, at least a portion of the totally reflected light back towards the measuring portion, whereby at least a portion of the light reflected back by the reflective portion undergoes an attenuated total reflection by the at least one measuring portion of the first surface,
decoupling the totally reflected light from the ATR prism,
detecting at least a portion of the light exiting the ATR prism.

As explained above, incoming infrared light is coupled to the ATR prism, where it undergoes twice an attenuated total reflection on the boundary between the measuring portion of the measuring surface of the ATR prism and the measured object. The light exiting the ATR prism may pass through further optical elements prior to being detected and subjected to further spectral analysis, as known in the art. The analysis of the obtained spectroscopic data may include a Fourier transform spectrometric analysis or any other suitable analysis.

The infrared spectroscopic measurements may be repeated for a plurality of different measurement sites or regions in and/or on the measured object. Thus, the method for infrared spectroscopy may include moving the measuring probe to a new region of the measured object and repeating the steps of bringing the at least bringing the at least one measuring portion in optical contact with the measured object, illuminating the at least one measuring portion, reflecting, by the at least one reflective portion of the reflective surface at least a portion of the totally reflected light (by means of attenuated total internal reflection) back towards the measuring portion, decoupling the totally reflected light (by means of attenuated total internal reflection) from the ATR prism, and detecting at least a portion of the outgoing light.

By repositioning the measuring probe (i.e. spatially moving the probe in a lateral and/or vertical direction), it is possible to conduct a multiple measurement at a plurality of spatially separated points within and/or on the measured object, so as to obtain a spatial scan, for example a two-or three-dimensional spatial spectroscopic scan of the object. The distance between the measuring points (i.e. the density of the spatial measurements) as well as their arrangement may be freely selected according to the specific application. Since the measuring probe and more specifically the ATR prism may be miniaturized, the spatial resolution of the scan may be very relatively high, for example in the sub-millimeter range down to two-digit micrometer range. In an example, the ATR prism may be rotated, in order to perform measurements at different annular areas or sectors around a particular measuring site, for example to obtain a 360° measurement data. In an example, at least a part of the optical elements for guiding light into and out of the ATR prism may be rotated as well.

The lateral spatial resolution (distance between two measuring points) may be about 0.5 to 2 mm (higher or lower distances are also possible). The lateral spatial resolution is generally limited due to diffraction effects. For example for a wavenumber of about 1000 cm⁻¹ (wavelengths of about 10 µm), the diameter of a spot of focused light in an ATR prism arrangement is about and greater than 100 µm, which imposes limits on the lateral spatial resolution of the measurements.

The depth resolution can be similarly varied and is generally dependent on the available measurement time (for example the available measuring time under surgery conditions). A depth resolution (i.e. a vertical resolution, for example in the direction of insertion of the microscope probe) may about 0.5 mm or even better, depending on the parameters of the employed light source, optical system and the available measuring time. For example, it may be possible to complete a measurement of a measurement of a three-dimensional volume of about 5 mm x 5 mm x 5 mm to about 30 mm x 30 mm x 30 mm with the above mentioned lateral and depth spatial resolution within 30 minutes. The movement and repositioning of the measuring probe to a new measurement point/site is preferably done by using a computer controlled robotized drive system. The robotized drive system may use information about the patient's form and position for precisely controlling the movement and positioning of the measuring probe.

Thus for example, if cancer is suspected, the affected organ (e.g. a prostate or a breast or a cervix of an uterus) may be spatially scanned, for example during surgery intervention. By repositioning the measuring probe to a new measuring site (by a suitable computer controlled drive system), it is possible to obtain a data set of infrared spectroscopic data at different points in one, two- or three-dimensional space, each measuring point being assigned a set of spectroscopic data. Preferably, the data set may be completed by further data obtained by other optical measurements, such as Raman spectroscopy, optical coherence tomography, microscopy, etc. The obtained (multidimensional) data set may subjected to statistical analysis, and based on the obtained data a classification in different grades of the measured tissue (benign to malign may be carried out.

The infrared spectroscopic measurement may be combined with further optical measurements, all of them using advantageously the same ATR prism. The method for infrared spectroscopy may comprise:
illuminating at least one portion of the first surface of the ATR prism that is in contact with the measured object with light for an additional optical measurement, wherein the light for the additional optical measurement is incident perpendicularly on the illuminated portion or at an angle deviating from the perpendicular incidence by no more than ±15°, preferably by no more than ±12°; and
detecting at least a portion of the light for additional optical measurement returned from the measured object.

Due to the normal or near normal incidence of the light for additional optical measurement on the illuminated portion of the measuring surface, the incident light for further optical measurement does not experience a total reflection, so that the light returned from the measured object may pass through the measuring surface and be directed towards a suitable detector. To guide measuring light for carrying out further optical measurement to the ATR prism and out of it, the apparatus for infrared spectroscopy may comprise at least one additional illumination and detection path (that may at least partially coincide). The at least one additional illumination and/or detection path may have further common components (other than the ATR prism) with the illumination and detection path, respectively for infrared spectroscopy.

The additional optical measurement (preferably label-free optical measurement) may include, but is not limited to, one or more of Raman spectroscopy, optical coherence tomography, swept-source spectral-domain optical coherence tomography measurements and microscopy.

For example, the infrared spectroscopic measurement may be combined with Raman spectroscopy measurement. The method may comprise illuminating at least one portion (measuring portion for Raman spectroscopy) of the measuring surface of the ATR prism with Raman excitation light, wherein the Raman excitation light is incident on the illuminated portion perpendicularly (normally) or at an angle deviating from the perpendicular (normal incidence) by no more than ±15°, preferably by no more than ±12°; detecting at least a portion of the Raman scattered light and subjecting the detected light to a Raman spectroscopy analysis. The Raman excitation light may be emitted by a suitable light source (for example a light source emitting light in the near infrared (NI), visible (VIS) and ultraviolet (UV) range) and be coupled into a Raman spectroscopy illumination path including the ATR prism.

It is also possible to combine the infrared spectroscopic measurement with optical coherence tomography measurements, in particular swept-source spectral-domain optical coherence tomography. The method may comprise illuminating at least one portion (measuring portion for OCT) of the measuring surface of the ATR prism with optical coherence tomography illumination light, wherein the optical coherence tomography illumination light is incident on the illuminated portion perpendicularly (normally) or at an angle deviating from the perpendicular (normal) incidence by no more than ±15°, preferably by no more than ±12°; detecting at least a portion of the light returned back from the measured object and subjecting the detected light to an optical coherence tomography analysis. The optical coherence tomography illumination light may be for example in the visible (VIS) or near infrared (NI) spectral range).

Still further it is possible to combine the infrared spectroscopic measurements with microscopic observation of the measured object, for example in the ultraviolet, visible spectral range or in near infrared spectral range. In this case the ATR prism and the measuring surface of the ATR prism may be used in the illumination and detection path of the microscopic system. This allows observations of the measured objects on a microscopic scale, while carrying out infrared spectroscopic measurements. Further it is also possible to carry out image processing on a microscopic scale, preferably in real-time, for example to control the progress of a surgical intervention. The method may comprise illuminating at least one portion of the measuring surface of the ATR prism with illumination light in the ultraviolet, visible or near infrared spectral range, wherein the illumination light is incident on the illuminated portion perpendicularly (normally) or at an angle deviating from the perpendicular (normal) incidence by no more than ±15°, preferably by no more than ±12°, detecting at least a portion of the light returned back by the measured object, thereby forming a microscopic image of the measured object.

In the above examples, it is possible to obtain data by a plurality of different optical measuring method from a substantially the same measuring site of the object. In this case the illuminated portion of the measuring surface for further optical measurement may overlap at least partially with the measuring portion for infrared spectroscopic measurement by double attenuated total reflection. However, it is also possible to provide an ATR prism, in which the different optical measuring methods use different portions of the measuring surface. For example, the measuring portion for the attenuated total reflection infrared spectroscopic measurement may not coincide with the area used for the additional optical measurements. Further, in the above examples, it is not necessary that the light used for the additional optical measurements uses the whole area of the measuring portion used by the attenuated total reflection infrared spectroscopic measurement.

The data from the Raman spectroscopy, optical coherence tomography, microscopic observation, etc. optimally complete the infrared spectroscopic data and allows, for example, considerably improved identification or classification of unknown objects. A combination with other optical sensors and optical measurement methods is also possible.

Further, it is possible to use the ATR prism also for guiding light (for example intense laser light) to a particular site of the object, for example to treat a tumor detected previously by using the ATR prism. The treatment light may for example be incident on the measuring portion of the measuring surface, so that no total reflection occurs. Thus, the light may pass through the measuring surface and reach the measured object, where it is absorbed. Accordingly, the method for infrared spectroscopy may further comprise illuminating at least one portion of the first surface of the ATR prism with light for treatment of the measured object (for example intense laser light), wherein the light for the treatment of the object is incident perpendicularly on the measuring portion or at an angle deviating from the perpendicular incidence by no more than ±15°, preferably by no more than ±12°. In an example, the portion illuminated with light for treatment of the measured object may at least partially overlap with or be within the measuring portion for infrared spectroscopic measurement by double attenuated total reflection, the measuring portion for Raman spectroscopy, the measuring portion optical coherence tomography and/or the portion illuminated with microscopic light.

The above and other objects, features and advantages of the present invention will become more apparent upon reading of the following detailed description of preferred embodiments and accompanying drawings. Other features and advantages of the subject-matter described herein will be apparent from the description and the drawings and from the claims. It should be understood that even though embodiments are separately described, single features thereof may be combined to additional embodiments.

### BRIEF DESCRIPTION OF THE DRAWINGS

FIG. 1 shows an exemplary apparatus for infrared spectroscopy;
Fig. 2 is an enlarged view of the ATR prism employed in the apparatus of Fig. 1;
FIGS. 3 to 5 illustrate the invariance of the average angle alpha_av from the individual incidence angles;
FIG. 6 shows an exemplary apparatus for infrared spectroscopy;
FIG. 7 shows an exemplary apparatus for infrared spectroscopy;

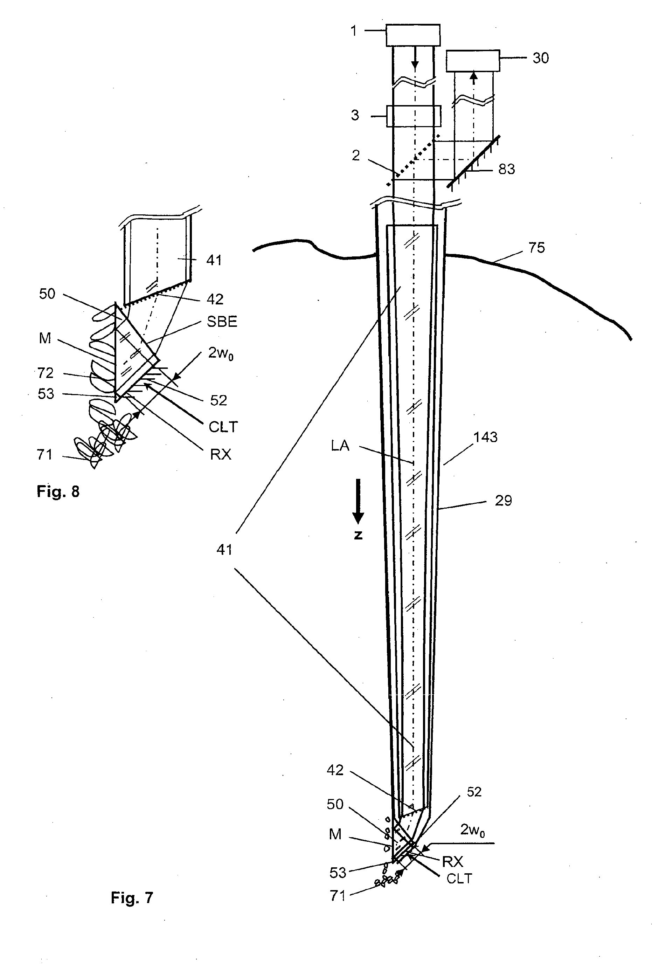

FIG. 7 shows an exemplary apparatus for infrared spectroscopy;
FIG. 8 is an enlarged view of the ATR prism employed in the apparatus of Fig. 7;

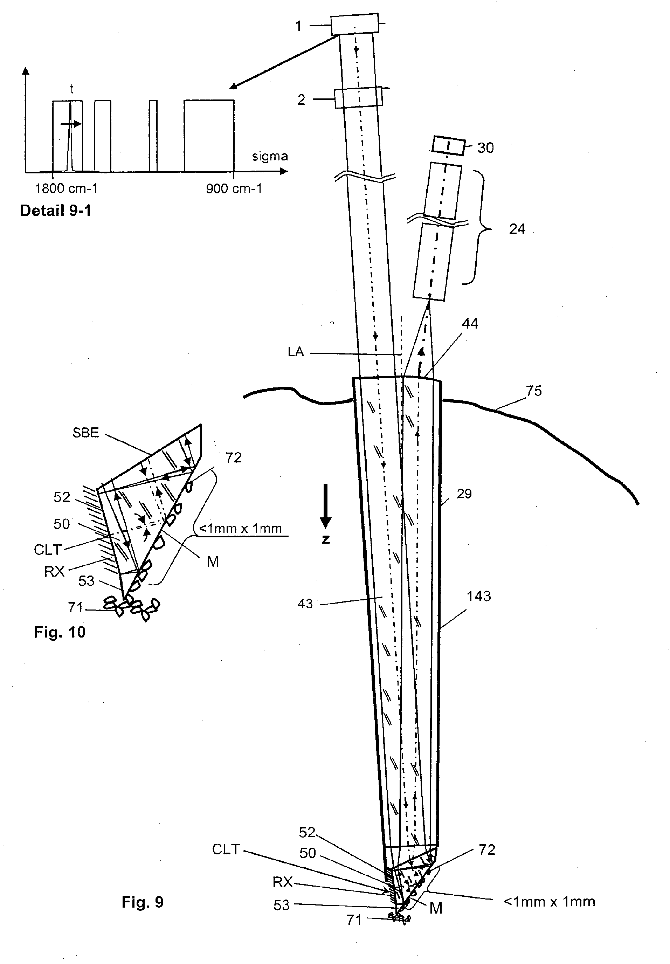

FIG. 9 shows an exemplary apparatus for infrared spectroscopy;
FIG. 10 is an enlarged view of the ATR prism employed in the apparatus of Fig. 9;

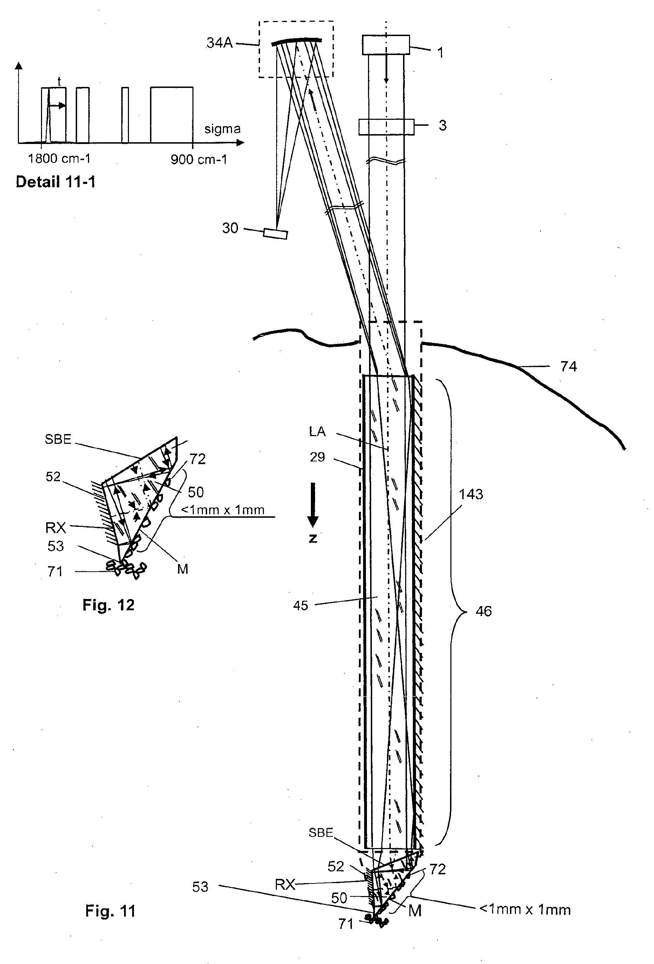

FIG. 11 shows an exemplary apparatus for infrared spectroscopy;
FIG. 12 is an enlarged view of the ATR prism employed in the apparatus of Fig. 11;

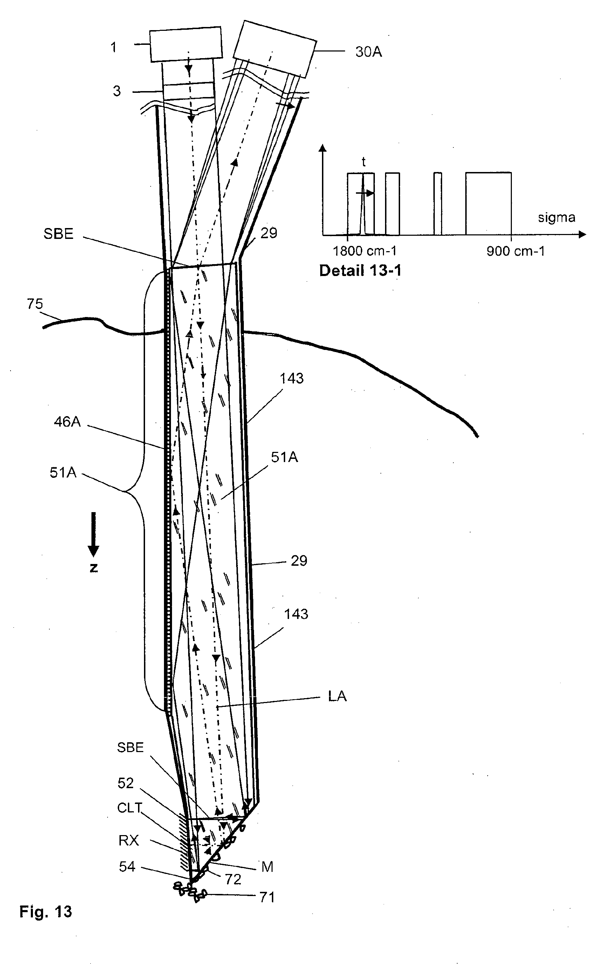

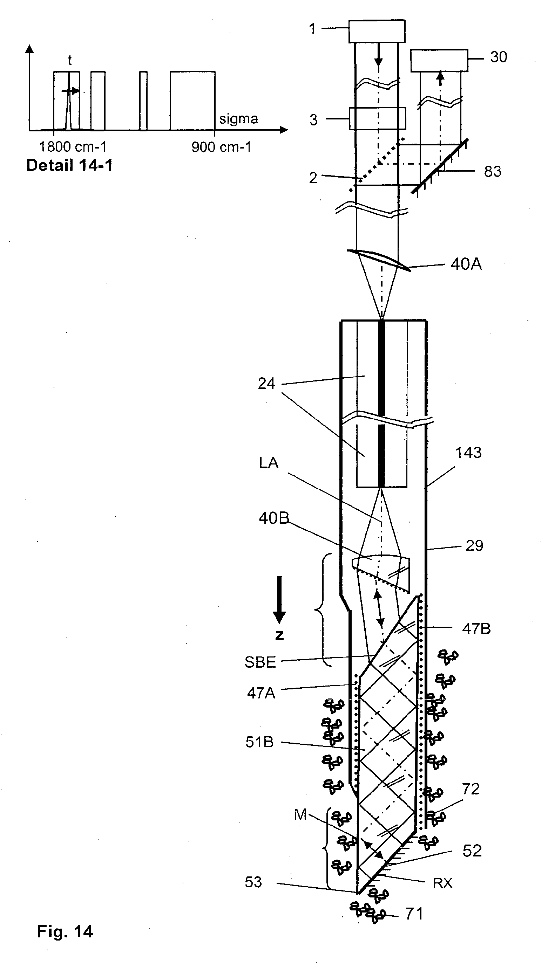

FIGS. 13 and 14 show exemplary apparatuses for infrared spectroscopy;

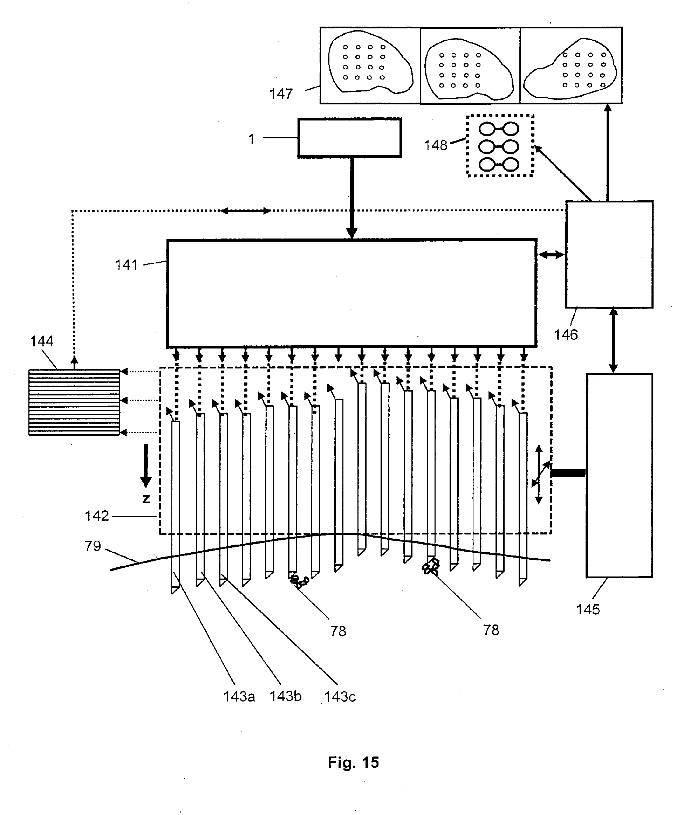

FIG. 15 shows a system for tumor diagnosis using an exemplary apparatus for infrared spectroscopy having a kit or a magazine of measuring probes;

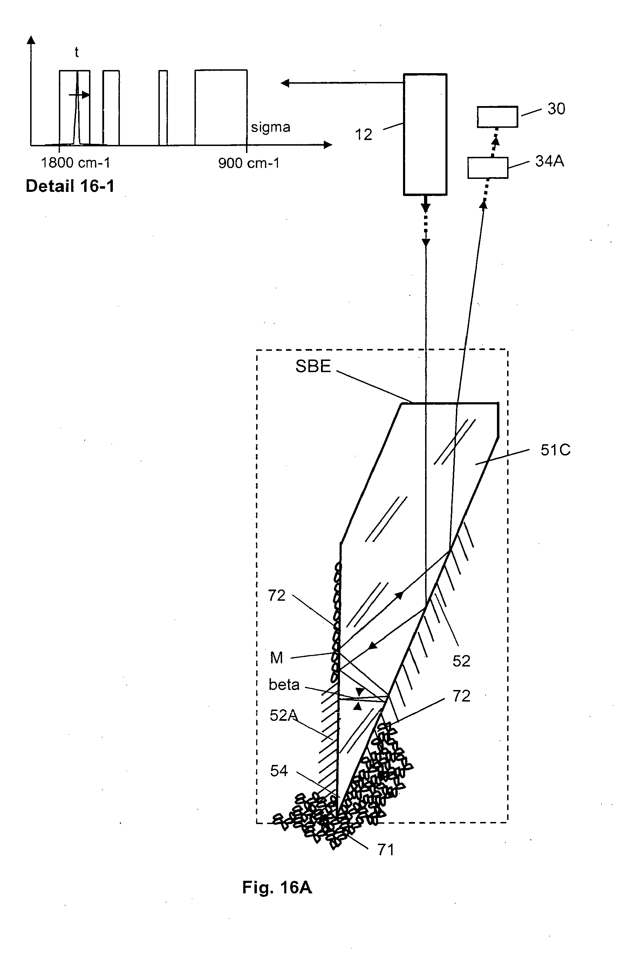

FIG. 16A shows an exemplary apparatus for infrared spectroscopy;

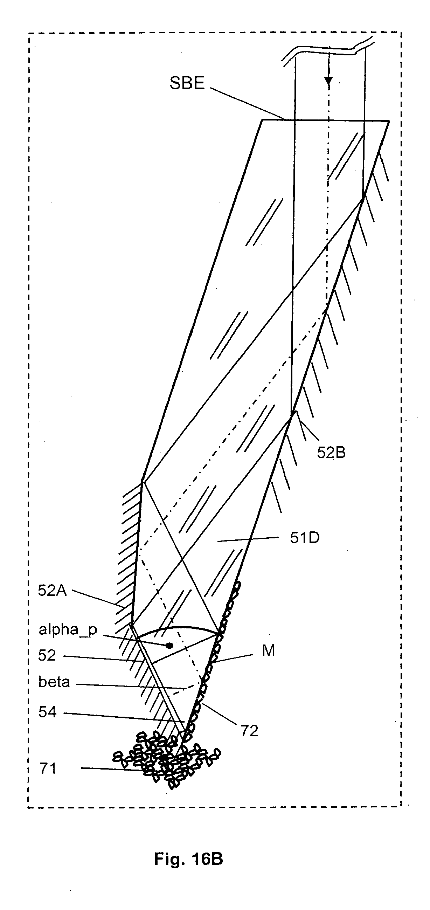

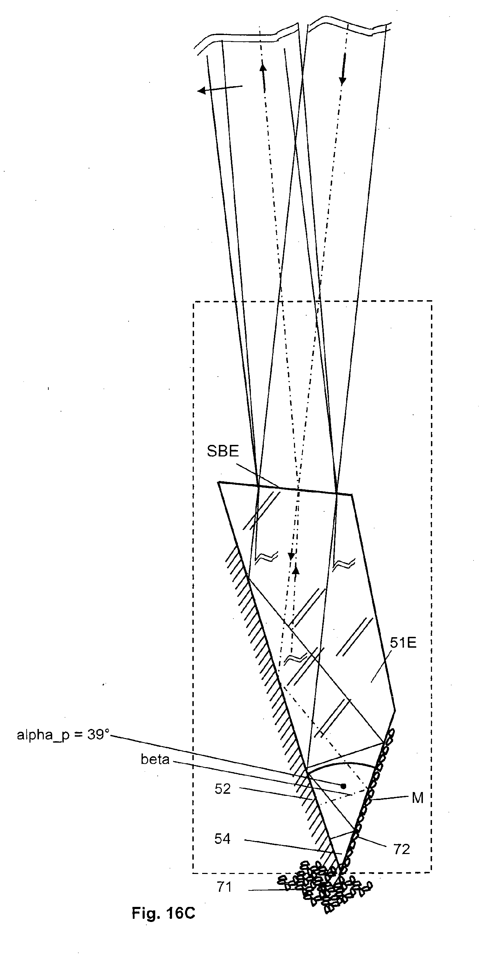

FIGS. 16B and 16C show exemplary ATR prisms that may be used for example in the apparatus shown in FIG. 16A;

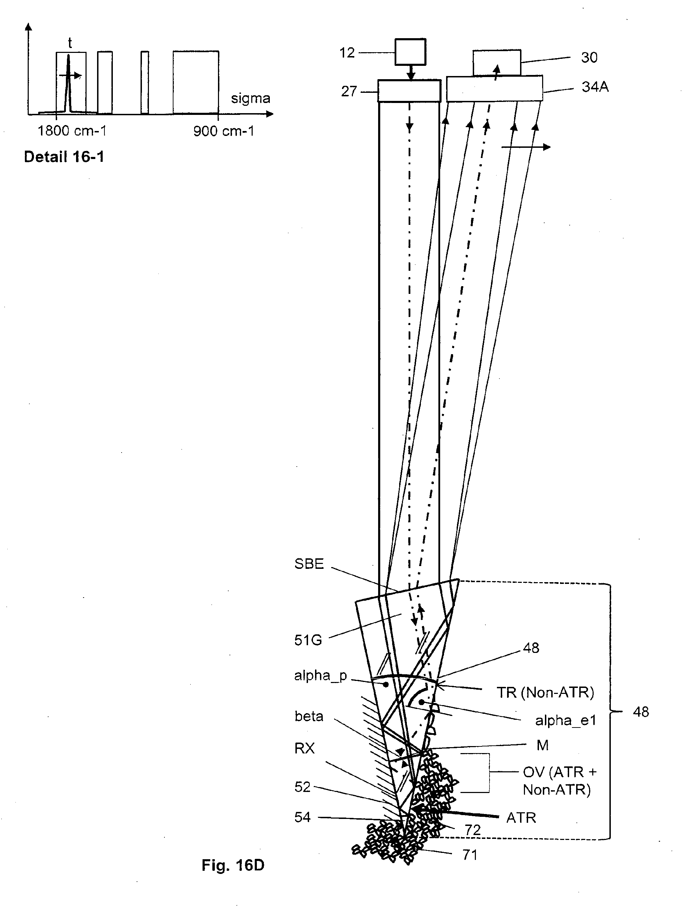

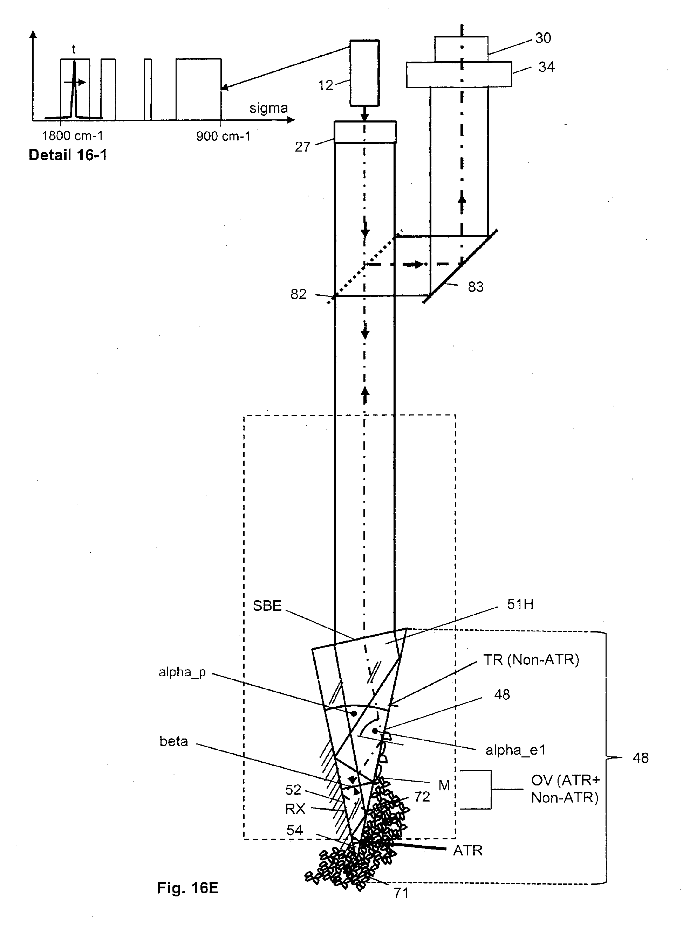

FIGS. 16D and 16E show exemplary apparatuses for infrared spectroscopy;

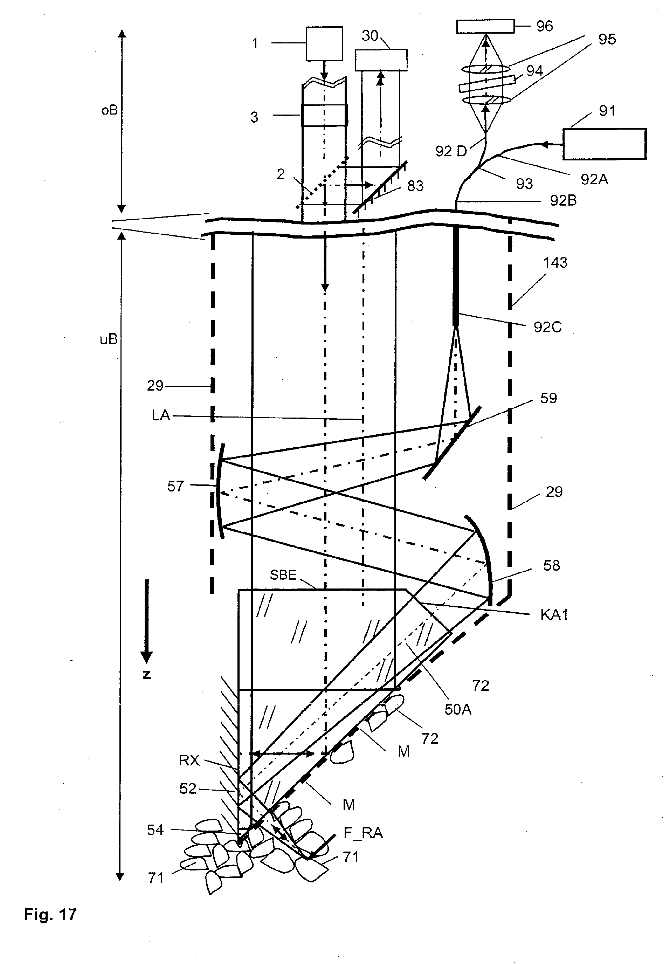

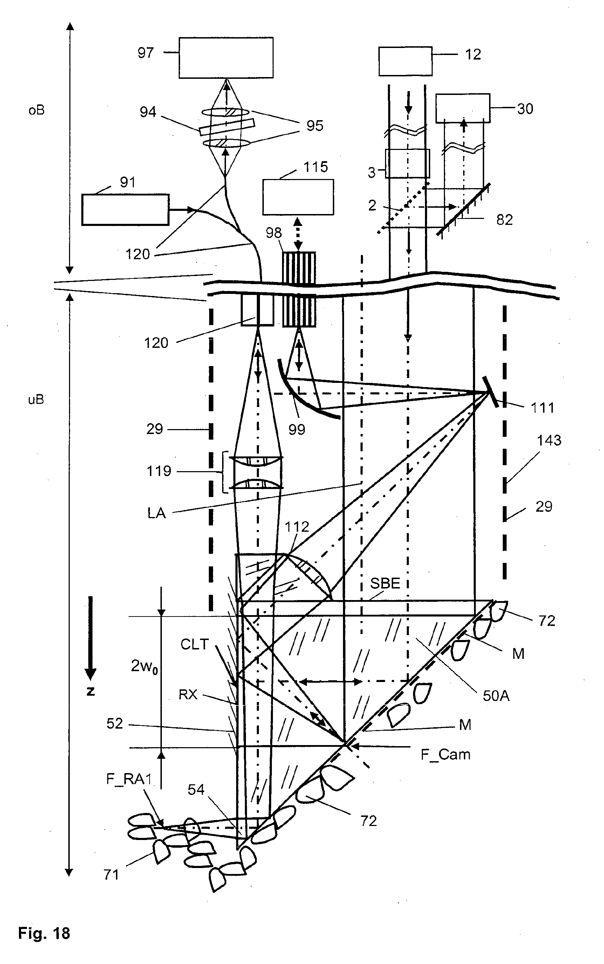

FIGS. 17 and 18 show exemplary apparatuses for infrared spectroscopy;

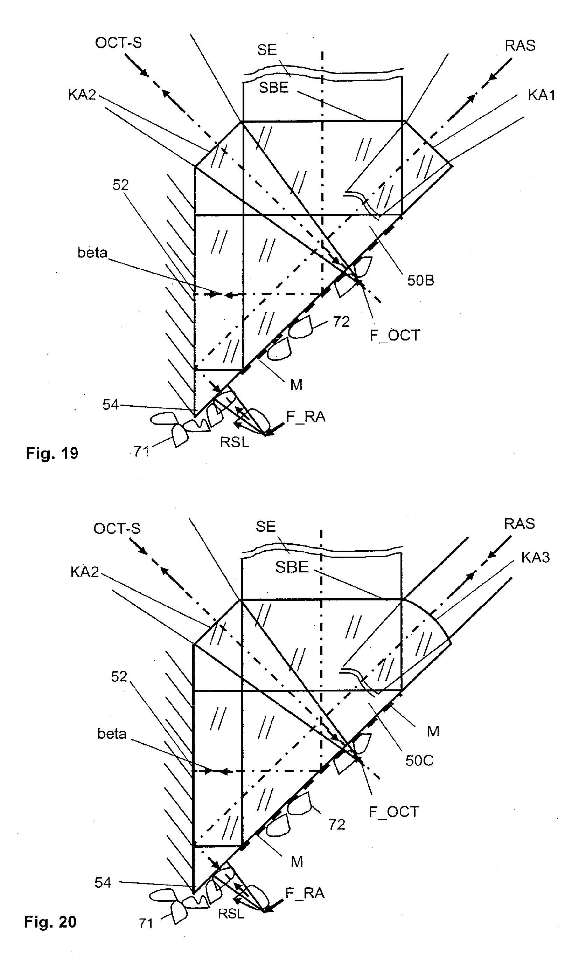

FIGS. 19 to 22 show exemplary ATR prisms for combining infrared spectroscopic measurements with additional optical measurements;

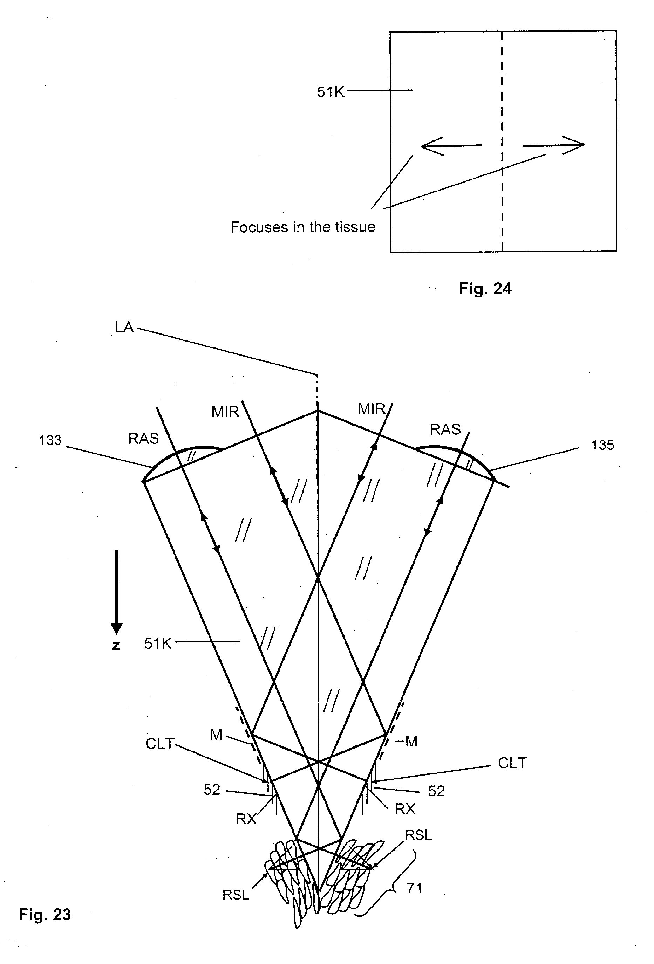

FIG. 23 shows an exemplary two-channel ATR prism;
FIG. 24 is a view from below of the two-channel ATR prism shown in FIG. 23;

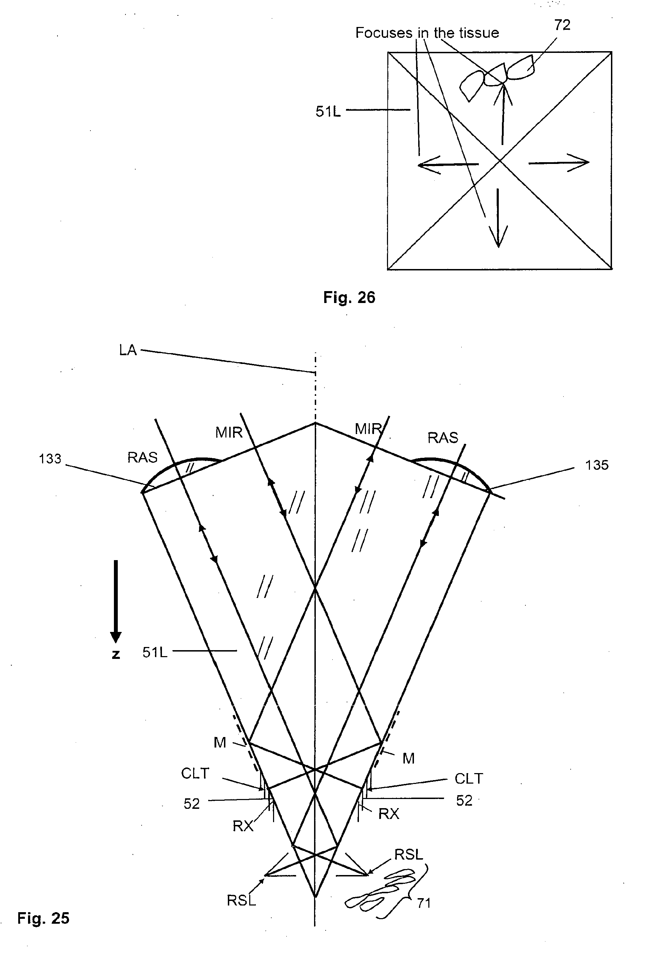

FIG. 25 shows an exemplary multi-channel ATR prism;
FIG. 26 is a view from below of the multi-channel ATR prism shown in FIG. 25;

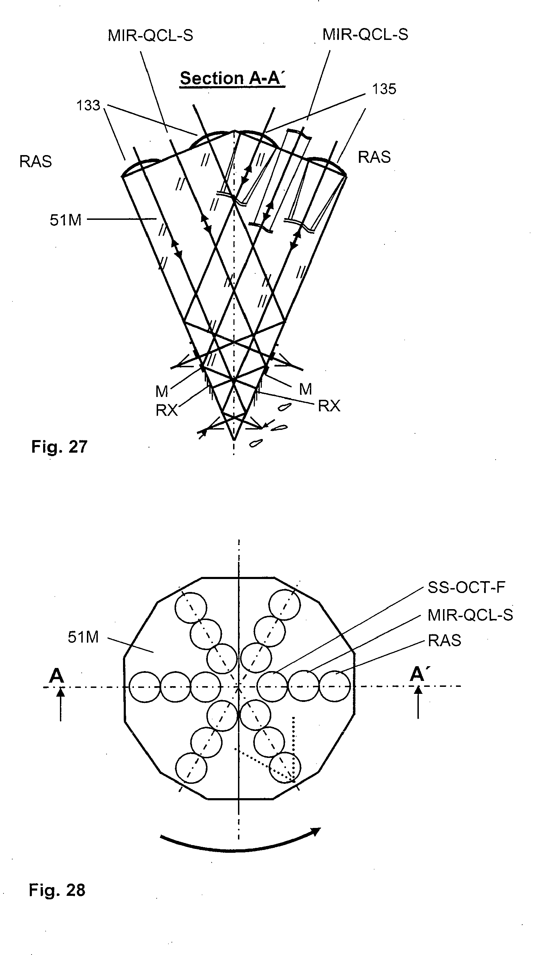

FIG. 27 shows an exemplary multi-channel ATR prism;
FIG. 28 is a view from below of the multi-channel ATR prism shown in FIG. 27;

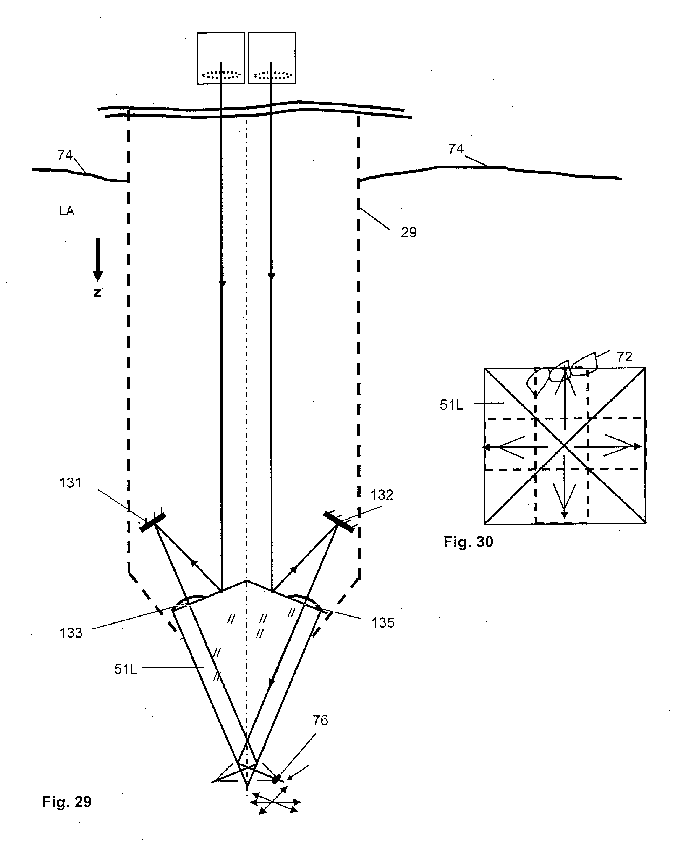

Fig. 29 shows the beam path of treatment light through an ATR prism (for example the ATR prism shown in Fig. 25);
Fig. 30 is a view from below of the ATR prism shown in Fig. 29.

### DETAILED DESCRIPTION OF THE INVENTION

Throughout the present application, the term "light" is used to mean any electromagnetic radiation from terahertz to deep ultraviolet range. The term "infrared light" is used to mean electromagnetic radiation in the near (NIR), mid (MIR) and far infrared range (FIR).

The figures are schematic representation, which are not up to scale and which may comprise parts that are enlarged or downsized for a better understanding. Further some parts may be omitted for better understanding.

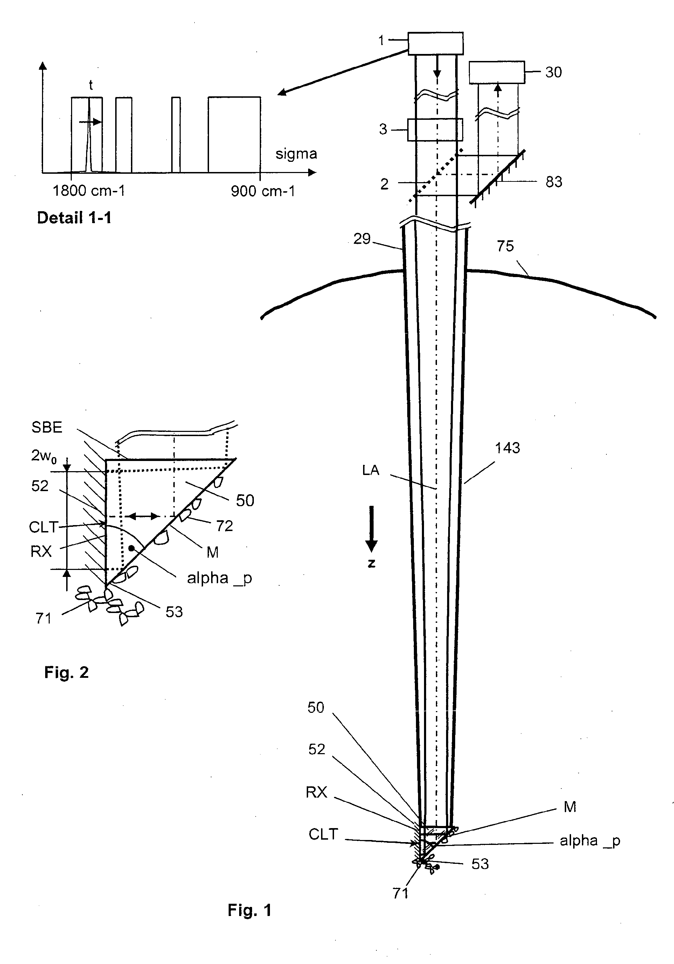

**Fig. 1** shows schematically the optical layout of an apparatus for infrared spectroscopy (infrared spectrometer) employing attenuated total internal reflection according to a first example. The apparatus may be used for cancer and/or metabolism diagnosis, for example for performing measurements in the depth of a breast. **Fig. 2** is an enlarged view of the ATR prism employed in the apparatus of Fig. 1. The measuring part M of the ATR prism may have a size equal to or smaller than 0.5 mm x 0.5 mm.

The apparatus for infrared spectroscopy comprises a quantum cascade laser battery 1 with a variable (scannable) wavelength as an infrared light source (so called swept source). The wavelength/wavenumber of the light emitted from the laser battery may be swept or scanned in time over a broad spectral range, for example from about 900 cm⁻¹ to 1800 cm⁻¹ (wavenumbers) corresponding to about 5.5 µm to 11.1 µm (wavelength). Preferably, it is possible to select out of the broad spectral range and optionally address (for example by amplitude modulation) individual spectral bands known as producing spectral information relevant for cancer and/or metabolism diagnosis. The infrared spectroscopic measurements are then carried out for the selected spectral bands. This reduces the radiation damage of the measured object and the overall measurement time. **Detail 1-1** shows symbolically the spectrum of the emitted infrared light with a number of selected spectral bands that are scanned with the time t.

The selected spectral bands are serially scanned in time (for example starting from the higher wavenumber in the direction of the lower wavenumbers), wherein the scanning spectral accuracy is preferably at least 1 cm⁻¹ and the spectral full width at half maximum of each scanned laser line is lower than 1 cm⁻¹.

The light emitted from the laser battery 1 is collimated by a suitable collimator that may be a part of the light source 1 and is coupled into a spectroscopic illumination path comprising a beam forming optical system 3, and a beam splitter 2. The beam forming optical system 3 forms a convergent light beam that is coupled into the measuring probe 143 and focused at or around the reflective portion RX of the reflective surface. In other words, the waist of the convergent light beam is imaged at or around the reflective portion RX. In Fig. 2, the center of the waist of the light beam is indicated as CLT and its diameter as 2w₀. The slight focusing of the incident light helps to miniaturize the ATR prism 50 and the measuring probe 143.

The measuring probe 143 has a stainless steel housing 29 and exhibits an elongated, substantially cylindrical or conical form. The measuring probe 143 may be formed as a hollow needle or a catheter with a maximal cross-section of about 2 mm x 2 mm, preferably about 0.6 mm x 0.6 mm. The longitudinal axis LA of the elongated measuring probe 143 may be generally parallel to the direction of insertion (z) of the measuring probe 143 into the measured object. In another example, the longitudinal axis LA may be at an angle to the direction of insertion of the measuring probe 143.

The measuring probe 143 may be connected to a suitable computerized mechanical driving mechanism (not shown in Fig. 1) that inserts the measuring probe 10 into the measured object (for example, a human or animal organ). To assist the insertion of the measuring probe 10 into the measured object, the measuring probe may be subjected to micro-vibrations, for example up and down vibrations in the direction of insertion of the measuring probe 10. The micro-vibrations may be ultrasonic micro-vibrations.

The measuring probe 143 comprises a first end (input) for coupling and decoupling the infrared light in and from the measuring probe and a second end at which the ATR prism 50 is arranged. In the example shown in Fig. 1 the incoming light is guided to the ATR prism 50 without the help of further optical elements arranged in the housing 29. However, it is also possible to arrange further optical elements in the housing as a part of the illumination and/or detection path. These further optical elements may for example comprise optical fibers, mirrors, lenses, prism elements and other suitable elements. Further, mechanical and/or electrical elements adapted to secure the ATR prism 50 in place and connect the measuring probe (mechanically and/or electrically) to a mechanical driving mechanism and/or a suitable controller may be arranged in the housing 29.

The ATR prism 50 is arranged at the second (front) end of the measuring probe 143. The ATR prism 50 is made of a diamond and has a diamond cutting portion (cutting blade) 53 at its front end for cutting through the tissue 71 of the examined organ. The ATR prism 50 has an optical coupling surface SBE, through which the illumination light for the infrared spectroscopy is coupled into the ATR prism 50 and through which the light is decoupled from the ATR prism 50 after interaction with the measured object. In this example, the optical coupling surface SBE is a plane surface, however it may also be a curved surface. The infrared light coupled through the first end of the measuring probe 143 is substantially normally incident on the plane optical coupling surface SBE. The ATR prism 50 has further a measuring surface (for example a plane surface) having a measuring portion M, which is in optical contact with the measured object, and a reflective surface with the reflective portion RX. The reflective surface is a plane surface that is arranged at a substantially right angle to the optical coupling surface. The reflective portion RX of the reflective surface is formed by applying a reflective layer 52 on the reflective surface. The reflective layer may be for example an aluminum layer. The reflective layer 52 may also exhibit a multilayer structure having preferably a hard coating layer as the outermost layer.

The measuring surface and the reflective surface intersect to thereby form a cutting portion 53 forming the tip of the measuring probe 143. The cutting portion 53 has a cutting angle of preferably about 39°. Thus, the ATR prism 50 has both an optical function (coupling infrared light into the measured object by means of attenuated total reflection) and a cutting function (cutting through the measured object, for example human tissue). The ATR prism 50 may be subjected to micro vibrations, in order to assist the cutting process and reduce the collateral tissue damage.

In the example shown in Fig. 1 the measured object is human tissue 71 that is cut by the cutting portion 53 immediately before the optical measurement upon insertion of the measuring probe into the tissue 71 of a living organ (such as prostate, lung, breast, etc.). While cutting through the observed tissue 71, a thin fluid film is formed on the measuring portion M. The presence of the thin fluid film improves the optical contact between the measuring portion M and the observed tissue. The part of the cut human tissue 71 that is in contact with the measuring portion M of the measuring surface is indicated by the reference numeral 72.

The prism angle alpha_p between the reflective surface and the measuring surface (and more specifically between the measuring portion M and the reflective portion R) is selected such that the light undergoes an attenuated total reflection by the measuring portion M and is reflected back to the measuring portion by the reflective portion RX, where it undergoes a second attenuated total reflection. Thus, the infrared light has twice an optical contact with the examined cut tissue 72: once prior and once after the reflection by the reflective portion RX. Preferably, the ATR prism 50 is configured such that prior and after the reflection by the reflective portion RX, the infrared light is incident on the substantially same part of the measuring surface (i.e. on the substantially same measuring portion or part of it). Thus, the infrared light is in optical contact with substantially the same portion of the examined tissue 71 both prior and after the reflection by the reflective surface. This contributes to enhancing the obtained measuring signal.

In the example shown in Fig. 1 the ATR prism is configured and arranged such that the light is incident on the optical coupling surface normally and the angle beta, which is the angle between the main ray of the light beam incident on the measuring surface M of the ATR prism 50 and the main ray of the exiting beam after the double total reflection at the measuring portion, is substantially 0°. In this case the average value alpha_av of the magnitudes of the two angles of incidence on the measuring portion M of the measuring surface prior to and after the reflection by the reflective portion RX, respectively (alpha_av = (alpha_e1 + alpha_e2)/2) is substantially equal to the prism angle alpha_p. For example, the critical total reflection angle is about 35° to 36° for an ATR prism of diamond (refractive index n = 2.4 for the middle infrared spectral range), which is configured for measurements of human soft tissue with a reference index of about 1.4 in the middle infrared range.

Preferably, the angle beta is greater than 0° and smaller than 12°. This may be achieved by configuring and arranging the ATR prism 50 such that the attenuatedly totally reflected light is incident on the reflective portion RX at an angle different from zero. In this case the angle beta is different from zero and may be configured to be smaller than 12°, preferably smaller than 10°, further preferably around 2°. A relatively large difference between the average value alpha_av and the critical total reflection angle contributes to reducing the sensitivity of the infrared spectroscopic arrangement with respect to angular deviations and not perfectly collimated incident light beams.

For infrared spectroscopic measurements, the measuring probe 143 with the ATR prism 50 is inserted through the skin surface 75 into the observed organ up to a desired measurement point. The soft organ tissue 71 is cut by the cutting portion 53, whereby a portion of the freshly cut tissue comes in contact with the measuring surface M (with a thin fluid film being formed between the measuring portion M and the tissue 71). An infrared spectroscopic measurement is carried out over the selected spectral ranges, each measurement comprising illuminating the measuring portion M with infrared light coupled into the ATR prism 50 through the coupling surface SBE (see Fig. 2), so that at least a portion of the light striking the measuring portion M undergoes an attenuated total reflection towards the reflective portion RX of the reflective surface and is back reflected by the reflecting portion towards the measuring portion M of the measuring surface, where it undergoes a second attenuated total reflection. After the second attenuated total reflection the infrared light is decoupled from the ATR prism 50 through the coupling surface SBE and directed in the detecting path comprising the detector 30. In the example shown in Fig. 1 the light exiting the ATR prism 50 takes essentially the same path as the incident light. The beam splitter 2 (that is part of both the illumination and detection path) separates the incident light from the exiting light. The detection path of the infrared spectrometer comprises further the mirror 83 and a detector 30. The detector 30 may be a spatially and/or spectrally integrally detecting MIR detector, such as a mercury-cadmium telluride (MCT) detector.

Based on the detected infrared spectroscopic data, it is possible to determine characteristics of the measured object (such as a molecular composition, etc.). The detected infrared spectroscopic data or derived characteristic features (for example by applying a Principle Component Analysis or other suitable statistical method) may be compared reference infrared spectroscopic data (or derived reference characteristic features) of various types of reference tissue samples (for example malign, tumor, healthy, etc. tissue samples). Thus it is possible to detect cancer or to obtain information about the metabolism in the observed organ. Preferably, the reference data is obtained on the basis of tissue samples from the same patient, said tissue sample having known properties (for example samples of surely healthy tissue and/or surely malign tissue identified as such by hystopathologic examination). The regions from which the reference tissue samples are obtained are preferably close to the observed (measured) regions.

Preferably, the measurement and data analysis are completed within short time, for example within a few minutes. The results of the infrared spectroscopic measurements may be presented to a surgeon and/or used in an automatic decision making process. The process of infrared spectroscopic measurement as well as individual optical and/or mechanical elements may be, as known in the art, computer controlled, thereby automating the whole measurement process.

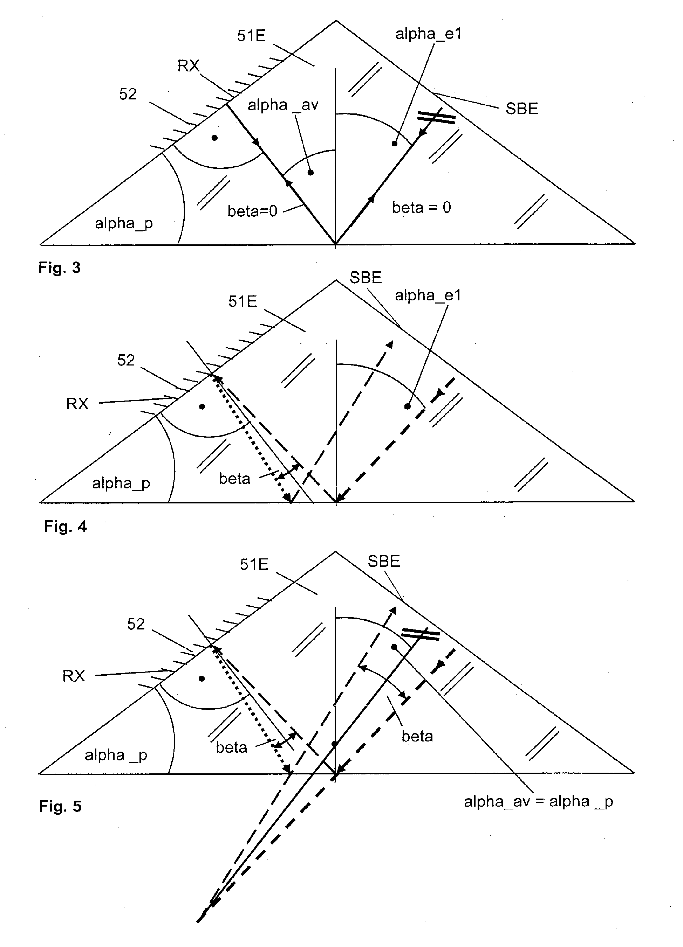

**Fig. 3 to 5** illustrate the invariance of the average value apha_av of the magnitude of the incident angles of the main ray of the light beam striking the measuring portion M prior to and after undergoing a reflection at the reflective portion RX with respect to the individual incident angles alpha_e1 and alpha_e2. The ATR prism is a monolith element with a fixed prism angle alpha_p between the measuring surface and the reflective surface (more specifically between the measuring portion M and the reflective portion RX).

**Fig. 3** illustrates the special case of normal incidence on the reflective portion RX of the reflective surface, wherein the angle of incidence (beta/2) of the attenuatedly totally reflected light beam on the reflective surface RX is zero. In this case the angle beta between the incoming light beam and the exit light beam is equal to zero (beta = 0).

**Fig. 4** illustrates the general case of light incidence on the reflective portion RX of the reflective surface at angles different from the angle of normal (perpendicular incidence). In this case, the angle of incidence beta/2 of the light beam on the reflective portion RX of the reflective surface is different from zero and alpha_e1 ≠ alpha_av.

**Fig. 5** illustrates the invariance of the average value alpha_av with respect to the individual incidence angles alpha_e1 and alpha_e2. The average value alpha_av is always equal to alpha_p (alpha_av = alpha_p), i.e. is invariant with respect to the individual angles of incidence alpha_e and alpha_12. This result in a high level of stability and robustness of the infrared spectroscopic measurements, even if the incident angle alpha_e 1 may vary to a certain extent, for example due to thermic or mechanical instability.

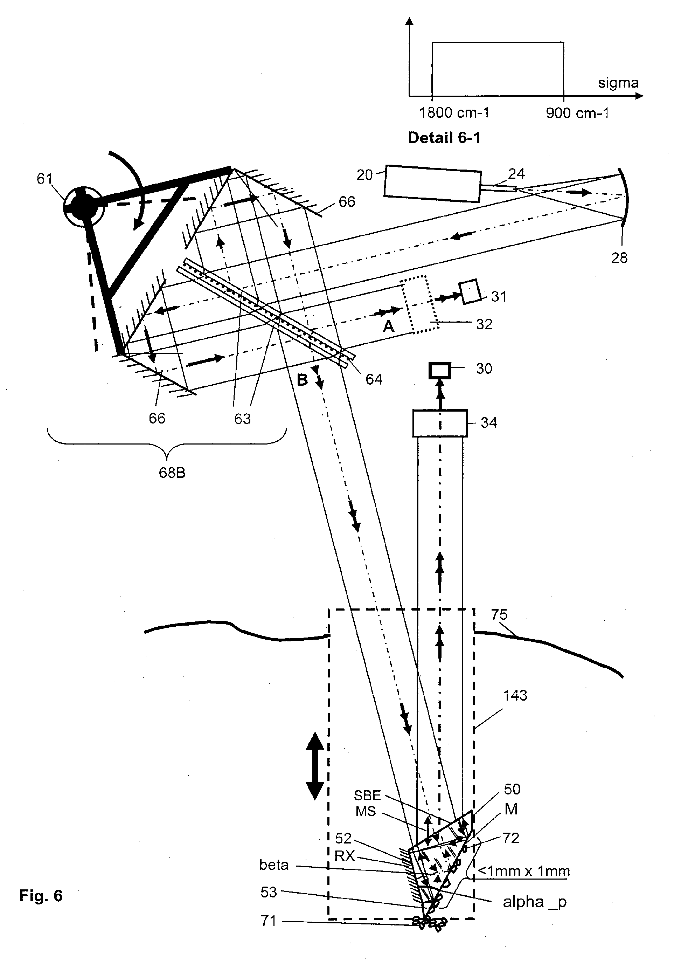

**Fig. 6** schematically presents the optical layout of an apparatus for infrared spectroscopy for cancer and/or metabolism diagnosis according to a second example. The apparatus for infrared spectroscopy of this example is a Fourier transform infrared spectrometer.

The light source is a broad band light source 20 (for example a quantum cascade laser battery) emitting light in the mid-infrared range from 900cm⁻¹ to 1800cm⁻¹ is used. The continuous spectrum of the emitted light is symbolically shown in **Detail 6-1.** The broad band light source 20 may have a fiber output for the infrared light. The emitted light is coupled into a silver halide fiber 24. The infrared light coming out of the silver-halide fiber is collimated by a collimating mirror 28 and enters an interferometer 68B comprising two triple-mirror type reflectors 66 and a beam splitter system comprising a beam splitter layer 64 sandwiched between two beam splitter plates 63. The interferometer 68B (which is a part of a Fourier transformation spectrometer with a spectral resolution between 2 cm⁻¹ and 4 cm⁻¹) comprises further a computer controlled drive system or actuator 61, e.g. a computer controlled rotational stage. The interferometric scan is realized by rotating the drive system or actuator 61, as shown in Fig. 6. An advantage of this type of rotating mirror type interferometer is that in its balanced state it is very robust with respect to vibrations due to environment influences. The interferometer is similar to the one disclosed in DE 30 05 520 A 1.

As in the example shown in Fig. 6, the interferometer 68B has two outputs. One output (output A) may be used to detect the spectrum of the illumination light and use it as a reference spectrum. To this extent, the interfering light coming from the output A of the interferometer 68B may be detected by a suitable detector 31 (for example a mercury-cadmium telluride (MCT) detector) associated with a corresponding optical system 32 and subjected to a Fourier analysis (for example FFT algorithm).

The interference light coming from the other output (output B) of the interferometer 68B may be used for infrared spectroscopic measurements by coupling it into the measuring probe 143 comprising an ATR prism 50. Unlike in the example shown in Fig. 1 and 2, the ATR prism 50 is arranged such that the incoming light beam and the exit light beam are angularly separated. The infrared light coupled from the first end of the measuring probe 143 is incident on the optical coupling surface SBE at an angle different from the angle of normal incidence. Further, the angle beta is different from zero and is around 4°. The prism angle is about 39°.

Preferably, the optical design is diffraction limited. The cross-section of the light spot at the measuring surface is about or smaller than 1 mm x 1 mm. The measuring portion M may be configured to be about 1 mm x 1 mm.

The light exiting the ATR prism 50 and the measuring probe 143 is detected by a suitable detector 30 (for example a mercury- cadmium-telluride detector (MCT)) with an associated detector optical system 34 and be subjected to further analysis (for example a Fourier analysis).

The ATR prism may be subjected to micro-vibrations MS to facilitate its insertion and cutting through the observed tissue 71.

The remaining elements and/or components and their arrangement are similar to those described in connection with the example shown in Figs. 1 and 2.

**Fig. 7** shows schematically the optical layout of an apparatus for infrared spectroscopy for cancer and/or metabolism diagnosis according to a third example. Fig. 8 is an enlarged view of the ATR prism employed in the apparatus of Fig. 7. The apparatus for infrared spectroscopy of this example is similar to the apparatus for infrared spectroscopy of the first example, with the following differences:
In the housing 29 of the measuring probe 143 there is positioned an elongated prism element 41 to guide the incoming infrared light to the ATR prism and guide the light exiting the ATR prism 50 out the measuring probe. The elongated prism element 41 may be made for example from ZnSe.

The elongated prism element 41 has a substantially plane light coupling surface (first face) which is arranged substantially perpendicular to the longitudinal axis LA of the measuring probe 143 and the main ray of the incoming infrared light beam. The second face at the other end of the elongated prism element 41 is at an angle to the longitudinal axis LA of the measuring probe 143 and is provided with a blazed diffractive grating 42. The blazed diffractive grating 42 is configured to at least partially compensate for the spectral dispersion in the elongated prism element 41, the ATR prism and/or other optical elements constituting the illumination and/or detection path. The ATR prism 50 (for example diamond ATR prism) is arranged at the front end of the housing 29, so that it is substantially enclosed by the housing 29, except for the measuring surface, which comes into contact with the measured object (in this example a tissue part 72 cut immediately before the measurement). The optical coupling surface SBE of the ATR prism is arranged at an angle to the face of the elongated prism element with the diffractive grating 42. Further, the infrared light beam (which is focused at or around the reflective surface) is incident at an angle to the optical coupling surface SBE.

The measuring surface having a measuring portion M is substantially parallel to the longitudinal axis LA of the measuring probe. The reflective surface having a reflective portion RX is arranged within the stainless steel housing 29, so that reflective surface is substantially covered by a side wall of the stainless steel housing 29. The reflective surface and the measuring surface intersect to form a cutting portion 53 having a cutting angle of 39°. As in the previous examples, the ATR prism 50 is configured such that there is a double attenuated total reflection at the measuring portion M. The remaining elements (such as beam forming optical system 3, beam splitter 2, mirror 83, detector 30, etc.) are similar to those described in connection with the first example.

**Fig. 9** shows schematically the optical layout of an apparatus for infrared spectroscopy for cancer and/or metabolism diagnosis according to a third example. **Fig. 10** is an enlarged view of the ATR prism employed in the apparatus of Fig. 9. The apparatus for infrared spectroscopy of this example is similar to the apparatus for infrared spectroscopy of the first example, with the following differences:

The light source 1 is a swept source with a scannable wavelength and selectable spectral bands, as disclosed in connection with the first example. **Detail 9-1** shows symbolically the spectrum of the emitted light. In this example, however, the beam forming optical system is omitted, so that the incoming light beam is rather well collimated.

In the housing 29 of the measuring probe 143 there is positioned an elongated prism element 43 to guide the incoming infrared light to the ATR prism and to guide light exiting the ATR prism (exit light) out of the measuring probe 143. The elongated prism element 43 may be made from ZnSe.

The elongated prism element 43 has a light coupling surface having a plane portion for coupling the incoming infrared light beam and a lens portion for focusing the light beam exiting the ATR prism, so that the focused exit light beam is coupled into an optical fiber 24 (for example a silver-halide optical fiber) and is guided to the detector 30. The substantially plane portion is arranged substantially perpendicular to the longitudinal axis LA of the measuring probe 143 and the main ray of the incoming infrared light beam. The second face of the elongated prism element 43 may be at an angle to the longitudinal axis LA of the measuring probe 143 and may be provided with a blazed diffractive grating (not shown).

The ATR prism 50 (for example diamond ATR prism) is arranged at the front end of the housing 29, so that it is partially enclosed by the housing 29. The optical coupling surface SBE of the ATR prism is arranged at an angle to the second face of the elongated prism element 43. The infrared light beam (which is focused at or around the reflective surface as in the first example) is incident at an angle to the optical coupling surface SBE.

The reflective surface is provided with a reflective portion RX formed by the reflective layer 52. The reflective surface is arranged at a slight angle to the longitudinal axis LA of the measuring probe. It is, however, possible to arrange the reflective surface, so as to be substantially parallel to the longitudinal axis of the measuring probe 143.

The measuring surface having a measuring portion M (approximately 1 mm x 1mm or smaller) is arranged at an angle the longitudinal axis LA of the measuring probe. The reflective surface and the measuring surface intersect to form a cutting portion 53 having a cutting angle of 30°. It is also possible to form the cutting portion not by the intersection of the reflective and measuring surface, but as an additional element (preferably also made of diamond) that is in fixed mechanical contact with the remaining parts of the ATR prism. In this case, the cutting surfaces of the cutting portion may be in flush with the measuring and reflective surfaces, respectively.

The ATR prism 50 is arranged such that the incidence angle of the incoming light beam at the optical coupling surface SBE and the angle beta are different from zero. As in the previous examples, the ATR prism 50 is configured such that there is a double attenuated total reflection at the measuring portion M.

The remaining elements are similar to those described in connection with the previous examples.

**Fig. 11** shows schematically the optical layout of an apparatus for infrared spectroscopy for cancer and/or metabolism diagnosis according to a third example. **Fig. 12** is an enlarged view of the ATR prism employed in the apparatus of Fig. 11. The apparatus for infrared spectroscopy of this example is similar to the apparatus for infrared spectroscopy of the first example, with the following differences:
The light source 1 is a swept source with a scannable wavelength and selectable spectral bands, as disclosed in connection with the first example. **Detail 11-1** shows the spectrum of the emitted light. In this example, however, the beam splitter 2 and the mirror 83 are omitted.

In the housing 29 of the measuring probe 143 there is positioned an elongated prism element 45 to guide the incoming infrared light to the ATR prism and guide the light exiting the ATR prism 50 out the measuring probe. The elongated prism element 45 is made of ZnSe. The prism element 45 has a reflective coating 46 (for example aluminum reflective coating). The reflective coating 46 is used in guiding the light beam exiting the ATR prism 50 out of the measuring probe 143. In particular, the elongated prism element 45 and the reflective coating 46 are arranged such as to angularly separate the incoming light beam from the exit light beam, so that the exit light beam may be guided via a suitable optical system 34A to the detector 30. The optical system 34A may comprise at least one curved mirror for imaging the infrared light beam onto the detector 30. The optical system 34A may have a chromatic correction, so that the lateral color spread is preferably reduced, to enable imaging onto a detector having a relatively small detector surface.

The elongated prism element 45 has a substantially plane light coupling face, that is substantially perpendicular to the longitudinal axis LA of the measuring probe 143. The incoming infrared light beam is substantially normally incident on the light coupling face, whereas the light exiting light beam is incident on the plane light coupling face at an angle.

The ATR prism 50 (for example diamond ATR prism) is arranged at the front end of the housing 29. The ATR prism 50 is similar to the ATR prism 50 of the example shown in Figs. 9 and 10 and is arranged in a similar manner. Further, the remaining elements (such as beam forming optical system 3, detector 30) are similar to those described in connection with the previous examples.

**Fig. 13** shows schematically the optical layout of an apparatus for infrared spectroscopy for cancer and/or metabolism diagnosis according to another example. The apparatus for infrared spectroscopy of this example is similar to the apparatus for infrared spectroscopy of the first example, with the following differences:
The light source 1 is a swept source with a scannable wavelength and selectable spectral bands, as disclosed in connection with the first example. **Detail 13-1** shows the spectrum of the emitted light. In this example, however, the beam splitter 2 and the mirror 83 are omitted.

The ATR prism 51A is an elongated prism element having a first, upper light guide part and a second, lower part having an optical and cutting function. The light guide part has an elongated, substantially rod-like form having a longitudinal axis that is substantially parallel to the longitudinal axis LA of the measuring probe 143. The lower part has a reflective surface providing with a reflective portion RX formed by the reflective layer 52 and a measuring surface having a measuring portion M that comes into contact with the measured object. The reflective surface is inclined with respect to the longitudinal axis LA of the measuring probe 143. The reflective portion RX and the measuring portion M intersect to thereby form a cutting portion 54. The cutting portion 54 need not be formed by the intersection of the measuring portion M and reflective portion RX, but may also be formed as an additional element (also made of diamond) having cutting surfaces in flush (in contact) with the measuring portion M and the reflective portion RX, respectively. Further, the reflective portion RX and the measuring portion may be arranged at a different position on the reflective and the measuring surface, respectively.

The elongated light guide part of the ATR prism is provided with a reflective coating 46A (for example aluminum reflective coating) on one of its sides. The elongated light guide part with the reflective coating is configured such as to angularly separate the incoming light beam from the exit light beam, so that the exit light beam may be guided to a detector assembly 30A, comprising a detector for infrared light (for example a mercury-cadmium- telluride detector), an achromatic optical focusing system and an optical fiber (such as a silver-halide fiber).

The arrangement of the reflective portion RX and the measuring portion of the ATR prism 51A is similar to those of the ATR prism 50 shown in Figs. 9 to 12. In particular, the reflective portion RX and the measuring portion are arranged so that the incoming light passing through the (substantially plane) optical coupling surface SBE undergoes a double attenuated total reflection at the measuring portion M, with an angle beta being (slightly) different from zero. Further, the remaining elements (such as beam forming optical system 3, beam splitter 2) are similar to those described in connection with the previous examples.

**Fig. 14** shows schematically the optical layout of an apparatus for infrared spectroscopy for cancer and/or metabolism diagnosis according to another example. This arrangement allows increasing the sensitivity of the spectroscopic measurements at the expense of the spatial resolution, since the measuring signals are collected from different spatial regions of the measured object.

The apparatus for infrared spectroscopy comprises a quantum cascade laser battery 1 with a variable (scannable) wavelength as an infrared light source emitting light in the spectral range of 900 cm⁻¹ to 1800 cm⁻¹. Similar to the infrared spectrometer shown in Fig. 1, it is possible to select and optionally address (by for example amplitude or phase modulation) individual spectral bands known to provide information relevant for the infrared spectroscopy measurements for cancer and/or metabolism diagnosis. **Detail 14-1** shows the spectrum of the emitted infrared light with a number of selected spectral bands.

The optical arrangement comprises a beam forming optical system 3, a beam splitter 2, a mirror 83 and a detector 30 for detecting infrared light. The components and their functions are substantially the same or similar to those described in connection with the previous examples. Further the infrared spectrometer comprises a coupling lens 40A (for example made of ZnSe) arranged upstream of the measuring probe 143. The coupling lens 40A is shared by the illumination and detection part of the infrared spectrometer. The coupling lens 40A couples the light beam into an optical fiber 24 (for example a silver-halide fiber) that is positioned at least partially within the stainless steel housing 29 of the measuring probe 143.

The incoming light coupled into the optical fiber 24 is guided to a lens prism 40B (e.g. a ZnSe lens prism) arranged within the housing 29. The lens prism 40B collimates the incoming light beam. The lens prism 40B is provided with a blazed diffractive grating on one surface thereof to at last partially compensate for the spectral dispersion of the optical system.

The ATR prism 50B may be made of diamond and has two side surfaces, an optical coupling surface SBE and a substantially plane reflective surface. The optical coupling surface SBE and the reflective surface are inclined (slanted) with respect to the side surfaces of the ATR prism 50B. The side surfaces of the ATR prism 50B are covered at least partially with reflective coatings 47A, 47B, respectively, thereby forming an optical guide portion. The reflective coating may be aluminum reflective coatings. The optical guide portion of the ATR prism 50B is arranged within the housing 29, wherein an opening is provided in the housing 29 at the position of the measuring portion M (not covered by the reflective coating 47A) provided in one of the side surfaces constituting the guide portion. The measuring portion M comes into contact with the measured object 72 to perform an infrared spectroscopic measurement by means of double attenuated total reflection.

The reflective surface has a reflecting layer 52 forming a reflective portion RX. The reflective surface is formed at the tip (front end) of the measuring probe 143 and connects the two side surfaces of the ATR prism 50B. A cutting portion 53 is formed by the intersection of one of the measuring surfaces and the reflective surface. It is, however, possible to form a cutting portion as a separate element integral with the remaining parts of the ATR prism 50B.

The ATR prism 50B is configured such that the collimated incoming light beam entering the ATR prism 50B through the optical coupling surface SBE is guided to the measuring portion by means of multiple reflections on the side surfaces and is incident on the measuring portion M, where it undergoes a double attenuated total internal reflection, as explained above in connection with the preceding examples. After the double attenuated total internal reflection, the infrared light goes back following substantially the same path as the incident light. The incoming and the exit light beams are separated by the beam splitter 2. The exit light beam is directed by means of a mirror 83 to the detector 30. Further, the remaining elements (such as beam forming optical system 3, beam splitter 2, mirror 83) are similar to those described in connection with the previous examples.

**Fig. 15** shows a system for in-vivo cancer diagnosis (preferably during surgical intervention). The system comprises an apparatus for infrared spectroscopy operating in the mid-infrared range of about 900 cm⁻¹ to 1800 cm⁻¹. The apparatus for infrared spectroscopy may be any of the apparatuses described in the present application. The apparatus comprises a kit or a magazine that includes a plurality of measuring probes 143a, 143b, 143c, ..., optionally with the associated optical systems. The number of measuring probes may vary, for example a kit or a magazine containing 16 measuring probes may be provided. The measuring probes may be arranged in blocks or matrices, for example in a 4 x 4 block 144.

The probes may supported rotatably and may be movable vertically (in the depth direction z) and/or horizontally. To this end, a suitable mechanical driving system 145, for example a robotized driving system that is controlled by a computer system 146, may be provided. In an example, each of the measuring probes may be independently rotated and/or moved in a vertically and/or horizontally. This allows a better adjustment to the form of the examined object, which may be determined by a suitable two-dimensional or three-dimensional measuring system (for example by means of triangulation) ahead of the spectroscopic measurement. Further, measurements at different annular areas around a particular measurement site may be carried out, for example to obtain a full 360° measurement at each site.

Thus, is possible to carry out multiple measurements in parallel by employing a plurality of measuring probes (for example of the same type) positioned at different spatial positions of the measured object (e.g. a human organ). For example, infrared measurements may be performed at 4 different positions that are rotated at 90° with respect to each other. Each measurement and the respective data analysis may be carried out within a couple of minutes and the overall measurement and analysis time preferably does not exceed 20 minutes. Thus for example, it is possible to obtain information about the spatial distribution of cancer or other malign tissue in an affected organ during a surgical intervention and use this information to support the decision of the surgeon, whether to incise a particular portion of the organ or not.

Alternatively, the measurements may be carried out serially, by selecting a different probe for each measurement. For example, it is possible to repeat the measurements using measuring probes specifically configured for measurements on different materials. Further, a contaminated measuring probe may be exchanged for a new one. Due to the invariance of the average value alpha_av, the alignment of the optical system after change of the measuring probe is relatively simple and may be carried out automatically.

The system for cancer diagnostics comprises further a display 147 for displaying the results of the infrared measurement (and optionally further information). The system may also comprise other display means 148, such as data googles or spectacles, a Head-up Display, a Head-Mounted-Display, etc.

**Fig. 16A** shows schematically the optical layout of an apparatus for infrared spectroscopy for cancer and/or metabolism diagnosis according to another example. The apparatus for infrared spectroscopy comprises a swept source light source 12 emitting light with scannable wavelength and preferably selectable spectral bands, as explained in connection with the example shown in Fig. 1. Detail 16-1 shows the spectrum of the emitted light. The apparatus for infrared spectroscopy includes further a detector 30 with an associated optical system 34A. The optical system 34A may be chromatically corrected to reduce the lateral chromatic spread. This allows the use of a detector with relatively small surface.

The ATR prism 51C employed in this example is fabricated as a relatively thin synthetic diamond plate, for example a diamond plate having a thickness of about 1.8 mm. The ATR prism has an optical coupling surface SBE, which is a substantially plane surface, and two surfaces, which intersect with each other, thereby forming a cutting portion 54 with a cutting angle of approximately 20° (alpha_p = 20°). Due to the sharp cutting portion 54 with small cutting angle, it is possible to easily insert the measuring probe 10 into the measured object and cut through the tissue 71 (for example a human organ).

One of the intersecting surfaces (measuring surface) comprises a measuring portion M configured to be brought into optical contact with the measured object 72. The measuring surface comprises furthermore a reflective portion formed by applying a reflective layer 52A on part of the measuring surface. The other one of the intersecting surfaces (reflective surface) comprises a reflective portion RX formed by a reflective layer 52. The ATR prism 51C is configured such that light incident on measuring portion M of the measuring surface undergoes a first attenuated total reflection and is guided back to the measuring portion M for a second attenuated total reflection by means of a reflection by the reflective layer 52, a reflection by the reflective layer 52A covering the reflective portion of the measuring surface and a second reflection by the reflective layer 52. Having undergone a second attenuated total reflection, the light is guided through to the optical coupling surface SBE by means of a reflection at the reflective surface.

The remaining components constituting the optical spectroscopic arrangement are the same or similar as in the first example.

**Fig. 16B** shows another ATR prism 51 D that may be used in any of the described apparatuses for infrared spectroscopy, for example in the one shown in Fig. 16A. The ATR prism 51 D is made of diamond and has two reflective surfaces, the first reflective surface being formed by the reflective layer 52 and the second reflective surface by the reflective layer 52A. The two reflective surfaces are formed at angle to each other. The measuring surface comprises a measuring portion M configured to be brought into contact with the measured object 72, where attenuated total reflection occurs. The measuring surface comprises further a reflective portion formed by the reflective layer 52B covering a part of the measuring surface. The measuring surface intersects with the first reflective surface, thereby forming a cutting blade 54 for cutting through the observed tissue 71.

The light passing through the optical coupling surface SBE is guided to the measuring portion of the measuring surface M by means of reflection at the reflective layer 52B of the measuring surface and a reflection at the reflective layer 52 or reflective layer 52A. There the incident light undergoes a first attenuated total reflection. The totally reflected light is guided back to the measuring portion for a second attenuated total reflection by means of a reflection by the reflective layer 52. Having undergone a second attenuated total reflection, the light is guided to the optical coupling surface SBE by means of a reflection at the reflective layer 52B of the measuring surface M and for some rays also by the reflective layer 52A. In the configuration shown in Fig. 16B the optical paths of the incident light beam entering the ATR prism 51 D and the light beam leaving the ATR prism 51D are substantially the same. Since the angle of incidence (for both the incoming and the exiting light) at the optical coupling surface is substantially zero or close to zero, the angular dispersion at the exit of the ATR prism 51 D is very low. The separation of the incoming and the exit light beam may be achieved by suitable optical means, for example a beam splitter (see for example Fig. 1).

It is, however, possible to configure the ATR prism 51 D such that the angle of incidence of the main ray of the incoming light beam on the optical coupling surface SBE is different than zero, so that the incoming light mean may be more easily separated from the exit light beam.

**Fig. 16C** shows another ATR prism 51 E that may be used in any of the described apparatuses for infrared spectroscopy, for example in the one shown in Fig. 16A. The ATR prism 51 E comprises a measuring surface and a reflective surface having a reflective portion formed by applying a reflective layer 52 on the reflective surface. The measuring surface and the reflective surface intersect to form a cutting portion 53 with a cutting angle, which is equal to the prism angle. In the example shown in Fig. 16C the prism angle is about 39° - 40°. The angle beta is about 2°. The ATR prism 51E exhibits relatively low angular dispersion at its output.

**Fig. 16D** shows schematically the optical layout of an apparatus for infrared spectroscopy for cancer and/or metabolism diagnosis according to another example. The apparatus for infrared spectroscopy may be any of the described apparatuses for infrared spectroscopy with the difference that it uses an ATR prism 51 G made of crystalline silicon (Si) having a high refractive index (n = 3.4) in infrared. Crystalline silicon has a high hardness and is biocompatible. Further, due to the high refractive index, ATR prisms with very sharp cutting blades having cutting angles of less than 30° may be provided. Such small cutting angles considerably improve the cutting through the measured object (for example a human organ). As explained above, the cutting function may be optionally further improved by subjecting the ATR prism 51G to micro-vibrations (for example ultrasonic vibrations).

Further, due its high refractive index, a crystalline silicon surface behaves almost as a perfect mirror when the angle of incidence to the surface is greater than 60°, save for a phase difference delta from approximately 55°. At such high incidence angles, total reflection without attenuation may be achieved. The mirror-like, non-attenuated total reflection may be advantageously used to guide incoming light coupled to the ATR prism 51G to a measuring portion M of a measuring surface in optical contact with the measured object 72 (for example freshly cut human tissue). In the example shown in Fig. 16D, the prism surface 48 has at least one portion where non-attenuated total reflection occurs. This portion behaves like a mirror/reflective portion.

The ATR prism 51G may be arranged such that the light beam strikes the measuring surface at a plurality of regions or parts under different angles of light incidence. The ATR prism 51 G may be configured such that in the upper part (identified as non-ATR in the figures), the light is incident at a high angle (for example about 60°), so that only non-attenuated total reflection occurs at the prism surface 48. In the lower part (identified as ATR in the figures), the light is incident at an angle at which attenuated total reflection occurs (for example, for an ATR prism made of crystalline silicon this angle is about 25°). The lower part of the measuring surface is in optical contact with the measured object 72 (e.g. freshly cut human tissue), so that information about the measurement object may be obtained by means of attenuated total reflection. In the middle part OV (overlapping region), both attenuated and non-attenuated total reflection occur. The diameter of this overlapping region depends for example on the incident angle alpha_e1. In the example shown in Fig. 16D, the middle part OV (i.e. the overlapping region) of the total reflection with and without attenuation) has a diameter of approximately 1 mm.

It is possible to advantageously use of the overlapping region OV to construct ATR prism with very small size (with respect to the thickness and the cross-sectional dimensions). For example, the cross-sectional size of the ATR prism may be reduced to match the size of the light beam. The light beam may be slightly focused, so that the angular spread of the light beam may be made as small as a few angular degrees. This enables forming an almost diffraction-limited spot of infrared light on the measuring surface and the miniaturization of the ATR prism and the measuring probe.

Due to the use of a miniaturized prism exhibiting nearly diffraction limited optical design, the damages to measured object (for example human organ or tissue) may be reduced, which makes the ATR prism particularly advantageous for in-vivo measurements in living organs.

More specifically, the apparatus for infrared spectroscopy shown in Fig. 16D comprises an infrared light source 12 in combination with a variable wavelength (for example a swept source type) light source, such as a quantum cascade light battery. Preferably individual spectral bands may be selected for performing the infrared spectroscopy measurements. **Detail 16-1** shows the spectrum of the emitted light. The light emitted from the light source is coupled into an optical system for adjustment of the beam's cross section 27 and is coupled to the ATR prism 51G via the optical coupling surface SBE.

The ATR prism 51 G is made of a crystalline silicon and has a plate-like form. The ATR prism 51G comprises an optical coupling surface SBE (a plane surface), a prism surface 48 having a measuring portion M and a reflective portion where non-attenuated total internal reflection occurs, and a reflective surface having a reflective portion RX formed by a reflective layer 52. The reflective surface and the measuring surface intersect, thereby forming a cutting portion 54. The prism angle alpha_p (which is also the angle of the cutting blade 54) is smaller or equal to 30°. The prism surface 48 having the measuring portion serves as a measuring surface.

After passing through the optical coupling surface SBE, the incoming light strikes the upper part of the surface 48 at an angle higher than 60° and is totally reflected. At such high incidence angle the surface 48 behaves like an almost perfect mirror and the total reflection is not attenuated (non-ATR). Accordingly, it is not necessary to employ additional reflective layer at this part, which reduces the costs of the optical system. Further, the ATR prism 51G is configured and arranged such that the angle of incidence at the measuring part M of the measuring surface 48 (including in particular the lower part ATR) is about 25°. At this incident angle, an attenuated total reflection is possible that may be advantageously used to obtain data about the measured object 72 in optical contact with the surface 48 at this part.

The ATR prism 51 G is so inclined, that at a prism angle alpha_p (angle between the measuring surface 48 and the reflective surface) of about 25°. The incident angle of the incoming light bean striking the optical coupling surface SBE is equal to or smaller than 10°, which assures sufficiently low spectral dispersion. The angle beta is equal to or lower than 2°, which assures a good coupling of the incoming and decoupling of the exit light beam.

After undergoing a double attenuated total reflection the light is guided out of the ATR prism 51 G by means of staggered reflections on the reflective surface and the non-ATR portion of the prism surface 48. The exit light beam enters a detection path comprising a detector 30 together with its optical system 34A. With such arrangement the spectral dispersion in the middle infrared range is relatively low. The elements constituting the illumination path and the detection path are similar to those described in connection with the previous examples.

**Fig. 16E** shows schematically the optical layout of an apparatus for infrared spectroscopy for cancer and/or metabolism diagnosis according to another example, similar to the one shown in Fig. 16D. The ATR prism 51 H is made of crystalline silicon, has a prism angle of approximately 25° (slightly above the critical total reflection angle) and is similar to the ATR prism shown in Fig. 16D. In Fig. 16E the angle beta is approximately 0° and the ATR prism 51H is arranged such that the incoming light and the exiting light follow generally the same optical path (i.e. the incoming light beam and the exit light beam are collinear). The two beams are separated by means of a beam splitter 82 and a mirror 83. Also in this prism the spectral dispersion is relatively low and has a little influence on the propagation of the light rays through the ATR prism.

In the above examples, the ATR prism is made of crystalline silicon (Si). However, the ATR prism may be made of other high refractive index materials, for example by crystalline germanium (Ge). The crystalline germanium may be produced by zone refining or floating zone process. As the refractive index of crystalline germanium (n = 4) is higher than the refractive index of crystalline silicon, the prism angle (and respectively the cutting angle) may be reduced to for example about 20° to 22° (the critical total reflection angle for human tissue with reference index of about 1.4 is about 20.5°). The small prism angle (cutting blade angle) improves the insertion of the probe in the measured object and respectively cutting through the object. Further, there is no need to apply a reflective layer on the measuring surface to the measuring surface. However, the crystalline germanium has a lower hardness than crystalline silicon and it may be less biocompatible.

**Fig. 17** shows an exemplary apparatus for infrared spectroscopy with an integrated Raman spectroscopy optical arrangement. The apparatus for infrared spectroscopy may be any of the described apparatuses for infrared spectroscopy. The optical arrangement is not shown up to scale, in particular the lower part uB is shown enlarged and the upper part oB downsized.

In addition to an infrared spectroscopy illumination and detection paths, the infrared spectrometer comprises a Raman spectroscopy illumination path and a Raman spectroscopy detection path. The Raman spectroscopy illumination path comprises a Raman excitation laser 91 (for example Nd:Yag-Laser emitting Raman excitation light with a wavelength of 1064 nm), a Y fiber coupler 93 that couples a monomode fiber 92A of the illumination path and a monomode fiber of the detection path 92D to a monomode fiber 92B outside the measuring probe, a monomode fiber 92C inside the housing 29 of the measuring probe, a mirror 59 (beam diverting mirror), a first off-axis parabolic mirror 57 and a second off-axis parabolic mirror 58. The mirror 59, the first off-axis parabolic mirror 57, the second off-axis parabolic mirror 58, the monomode fibers 92B and 92C and the Y coupler 93 are also part of the Raman spectroscopy detection path which further comprises an optical transfer system 95 (comprising for example two lenses), a notch filter 94 and a Fourier spectrometer for spectrally analyzing the scattered Raman light 96. In an example, the notch-filter blocks the Raman excitation light with wavelength 1064 nm emitted from the laser 91.

The monomode fiber 92B confocally discriminates the light returned from the measured object and thus eliminates the portion of the (non-Raman) scattered light during Raman spectroscopy measurement.

The first off-axis parabolic mirror 57 and/or the second off-axis parabolic mirror 58 may be configured to correct or at least partially compensate the overall aberrations in the optical path. In an example, the mirror 59 may include an aberration corrective function to correct or at least partially compensate for the overall aberrations in the optical path. The mirror 59, the first off-axis parabolic mirror 57, the second off-axis parabolic mirror 58 and optionally any further optical elements may be integrated within the housing 29 (for example a stainless steel housing) of the measuring probe.

The ATR prism 50A (for example a diamond ATR prism) is used as an optically coupling element for both infrared spectroscopy and Raman spectroscopy. To couple the Raman excitation light into the ATR prism 50A and decouple the scattered Raman light returned from the measured object, the ATR prism exhibits a second optical coupling surface KA1. The second coupling surface may be a plane surface or a curved surface (for example a convex or concave surface). The measuring surface M of the ATR prism 50A serves as a surface for coupling both the totally reflected evanescent light for infrared spectroscopy and Raman excitation light into the measured object 72.

The Raman excitation light is focused at point F_RA. The scattered Raman light returned from the measured object enters through the measuring surface into the ATR prism 50A. The ATR prism 50A comprises further a reflective surface having a reflective portion RX formed by a reflective layer 52, which directs the totally reflected light back to the measuring portion M, where it undergoes a second total reflection. It is thus possible to carry out substantially simultaneously and at substantially the same measuring site both infrared spectroscopy measurements and Raman spectroscopy measurements.

In the example shown in Fig. 17, the incoming light for infrared spectroscopy measurement is incident on the optical coupling surface SBE of the ATR prism 50A substantially normally. Further, the ATR prism 50A is configured such that the angle beta is approximately zero. The incoming light is separated from the exiting light for the infrared spectroscopy measurement by means of a beam splitter 2 and a mirror 83.

Instead of providing an infrared spectrometer having two separate measuring paths (one for the infrared spectroscopy measurements and one for the Raman spectroscopy measurements) it is possible to use a single two-channel Fourier spectrometer, the first channel being used for detection and analysis of the Raman scattered light (such as NIR Raman scattered light) and the second channel being used for infrared spectroscopy measurements. It is also possible to employ a broadband one-channel Fourier spectrometer with CAF₂- components for both NIR Raman scattered light analysis and for infrared spectroscopy analysis up to a wavenumber of about 1100 cm⁻¹, in combination with an infrared broadband laser and integration of the Raman spectroscopy measuring path and the infrared spectroscopy measuring path.

Further, instead of having a Raman spectroscopy measuring path, the infrared spectrometer may have an optical coherence tomography measuring path, a microscopic measuring path, etc. Thus, the second optical coupling surface KA1 of the ATR prism may be configured for coupling optical coherence tomography light (in particular swept-source spectral-domain OCT), microscopic light in the visible region, etc..

The infrared spectrometer may integrate a plurality of further optical sensors or optical image forming or measuring systems, such as for example the above mentioned Raman spectroscopy sensors, OCT sensors (in particular swept source OCT sensors), microscopic observation, etc. The Raman spectroscopy sensors and the OCT sensors may operate in the near infrared spectral range and the microscopic observation may be carried out in visible range. The ATR prism may be dimensioned accordingly and comprise further optical coupling surfaces. All sensors may use the same measuring surface of the ATR prism (which is in optical and preferably mechanical contact with the measured object) to couple the respective measurement light into the measured object.

**Fig. 18** shows an exemplary multi-sensor apparatus for infrared spectroscopy with an integrated Raman spectroscopy optical arrangement and a microscopic arrangement. The apparatus for infrared spectroscopy may be any of the described infrared spectrometers. The optical arrangement is not shown up to scale, in particular the lower part uB is shown enlarged and the upper part oB downsized.

In addition to an infrared spectroscopy illumination and detection paths, the multi-sensor infrared spectrometer comprises a Raman spectroscopy illumination and detection paths as well as microscopy illumination and detection paths.

The Raman spectroscopy illumination path comprises a Raman excitation laser 91 (for example Nd:Yag-Laser emitting Raman excitation light with a wavelength of 1064 nm), a Y fiber coupler that couples a monomode fiber 120 of the illumination path and a monomode fiber 120 of the detection path to a common monomode fiber 120 reaching inside the housing 29 of the measuring probe 143 and an optical coupling system 119 comprising two lenses. The optical coupling system and the Y coupler are also part of the Raman spectroscopy detection path which further comprises an optical transfer system 95 (comprising for example two lenses), a notch filter 94 and a dispersive one-channel Fourier spectrometer 97 for spectrally analyzing the scattered Raman light. In an example, the notch-filter blocks the Raman excitation light with wavelength 1064 nm emitted from the laser 91. The monomode fiber 120 confocally discriminates the light returned from the measured object and thus eliminates the portion of the (non-Raman) scattered light during Raman spectroscopy measurement.

The microscopic illumination path comprises the illumination/camera assembly 115. The microscopic detection path comprises a collimating lens 112, a mirror 111, an off-axis ellipsoid mirror 99, an optical waveguide 98 and an illumination/camera assembly 115. The light source 12 in this example corresponds to the light source 1 in the first example. Further, the infrared spectrometer detection and illumination paths correspond generally to the illumination and detection paths of the infrared spectrometer according to the first example.

The ATR prism 50A (for example a diamond ATR prism) is used as an optical coupling element for all three optical measurements: infrared spectroscopy, Raman spectroscopy and microscopy observation. The light for all three optical measurements is coupled and decoupled through the same optical coupling surface SBE (which in this example is a plane surface). The measuring surface M of the ATR prism 50A also serves as a surface for coupling both the totally reflected evanescent light for all three optical measurements into the measured object 72.

The Raman excitation light is focused at point F_RA1. The microscopic observation light is imaged/focused at point/area F_Cam. The dashed line indicates the measuring area used for the infrared spectroscopic light. The ATR prism 50A comprises further a reflective surface having a reflective portion RX formed by a reflective layer 52, which directs the totally reflected light back to the measuring portion M, where it undergoes a second attenuated total reflection. The reflective portion RX also directs the microscopic illumination light towards the point/area F_cam. It is thus possible to carry out substantially simultaneously and at substantially the same measuring site three different optical measurements and observations.

**Fig. 19** shows an exemplary ATR prism 50B (for example a diamond ATR prism) which is configured to serve as an optical coupling element for infrared spectroscopy, Raman spectroscopy and optical coherence tomography measurements.

The ATR prism 50B has a first optical coupling surface SBE for coupling and decoupling light for the infrared spectroscopy, a second optical coupling surface KA1 for coupling and decoupling light for the Raman spectroscopy and a third optical coupling surface KA2 for coupling and decoupling light for the optical coherence tomography measurements. The first coupling surface SBE, the second coupling surface KA1 and the third coupling surface KA2 are substantially plane surfaces.

The measuring surface serves as a surface for coupling all three light beams (i.e. the light beam undergone an attenuated total reflection, the Raman excitation light beam and the optical coherence tomography light beam) into the measured object 72. The three different optical measurements may use different or partially overlapping parts of the measuring surface or measuring portion. In the example shown in Fig. 19 the Raman excitation light is focused at point F_RA and the optical coherence tomography light at point F_OCT. The dashed line on the measuring surface indicates the measuring portion M used for the attenuated total reflection

(i.e. for the infrared spectrometry measurement). The beam path of infrared spectroscopy is indicated as SE, the beam path of the Raman spectroscopy as RAS and the beam path of the optical coherence tomography as OCT-S.

The ATR prism 50B comprises further a reflective surface RX having a reflective portion formed by a reflective layer 52, which directs the light undergone a first attenuated total reflection back to the measuring portion M, where it undergoes a second attenuated total reflection.

With the ATR prism 50B shown in Fig. 19 it is possible to carry out substantially simultaneously and at substantially the same measuring site of the measured object 72 infrared spectroscopy measurements, Raman spectroscopy measurements and OCT measurements.

In the above example, it is possible to couple near infrared light for microscopic observation in the near infrared spectral range via the second optical coupling surface KA1 or the third optical coupling surface KA3. The microscopic measurement path may be at least approximately parallel to the OCT measurement path or the Raman spectroscopy measurement path.

**Fig. 20** shows another exemplary ATR prism 50C (for example a diamond ATR prism) configured to serve as an optical coupling element for infrared spectroscopy, Raman spectroscopy and optical coherence tomography measurements. The ATR prism 50C of this example generally corresponds to the ATR prism 50B shown in Fig. 19 with the difference that the coupling surface KA3 in the optical path RAS of the Raman spectroscopy is a curved surface with an optical power.

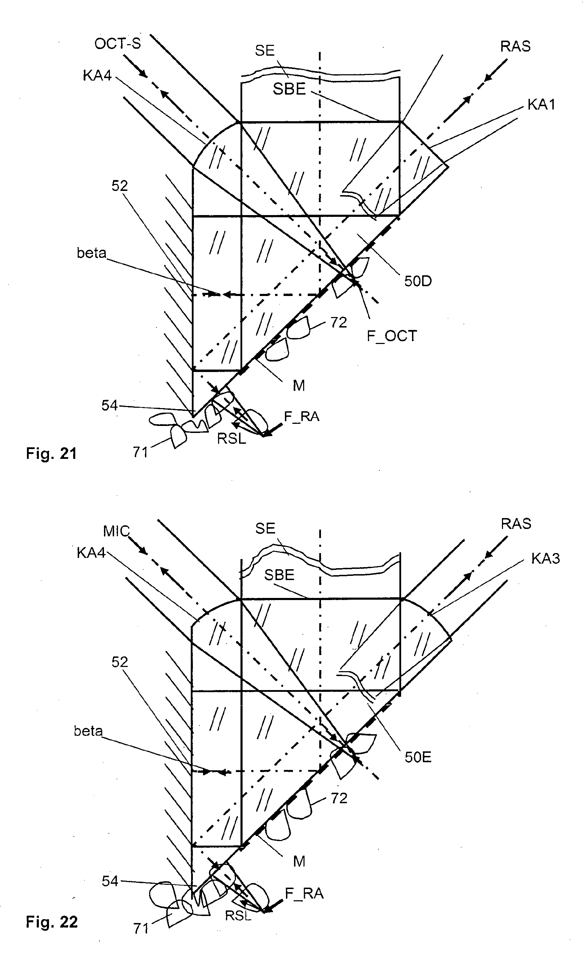

**Fig. 21** shows another exemplary ATR prism 50D (for example a diamond ATR prism) configured to serve as an optical coupling element for infrared spectroscopy, Raman spectroscopy and optical coherence tomography measurements. The ATR prism 50D of this example generally corresponds to the ATR prism 50B shown in Fig. 19 with the difference that the coupling surface KA4 in the optical path OCT-S of the optical coherence tomography (in particular swept source spectral domain optical coherence tomography) is a curved surface with an optical power.

**Fig. 22** shows another exemplary ATR prism 50E (for example a diamond ATR prism) configured to serve as an optical coupling element for infrared spectroscopy, Raman spectroscopy and microscopy measurements or observations. The ATR prism 50E of this example generally corresponds to the ATR prism 50B shown in Fig. 19 with the difference that instead of a coupling surface for optical coherence tomography, the ATR prism has a coupling surface for microscopic observations. Further both the coupling surface KA3 in the optical path RAS of the Raman spectroscopy and the coupling surface KA4 in the optical path MIC of the microscopic observations are curved surfaces with an optical power.

In all of the examples shown in Figs. 19 to 22, the ATR prism is arranged such that the angle of incidence of the main ray of the light beam for Raman spectroscopy and/or optical coherence tomography and/or microscopic observations at the measuring portion is zero or near zero. Preferably, the angle of incidence is lower than 12°, for example several angular degrees. One or more of the optical coupling surfaces KA1 to KA4 may be a plane or a curved surface, for example a convex curved surface to introduce optical power in the respective optical path. The employment of a curved optical coupling surface may be advantageous, in particular in terms of miniaturization of the optical path for the Raman spectroscopy, optical coherence tomography, in particular swept-source spectral-domain OCT or microscopy.

**Fig. 23** shows an ATR prism 51K made from diamond with two measuring channels for the infrared spectroscopy measurement and two additional channels for Raman spectroscopy. **Fig. 24** shows a view from the bottom of the ATR prism shown in Fig. 23 (i.e. viewed from the side of the apex of the pyramid ATR prism). The optical path of the infrared light for spectroscopic measurement is indicated as MIR. The optical path of the Raman spectroscopic light as RAS.

The ATR prism 51 K has a pyramid form having a longitudinal axis LA. The ATR prism 51 K comprises two optical coupling surfaces for coupling infrared light in the prism and decoupling infrared light out of the ATR prism 51 K, each optical coupling surface being assigned to a different measuring channel. Focusing lenses 133 and 135 are provided on the optical coupling surfaces, respectively (one focusing lens on each optical coupling surface).

The ATR prism 51 K comprises further a first side surface and a second side surface intersecting with each other at the apex of the pyramid and forming a double sided cutting portion. The first and the second side surfaces each have a measuring portion M that is in optical contact with the measured object (e.g. freshly cut tissue) and a reflective part RX formed by a reflective layer 52. Infrared light passing through the first one of the optical coupling surfaces (at part where the lens 133 or 135, respectively is not arranged) undergoes an attenuated total reflection at the measuring portion M of the first surface, is reflected back by the reflective portion RX of the second side surface towards the first side surface, strikes again the measuring portion M of the first side surface and undergoes a second attenuated total reflection, to thereby form a first exit light beam (first measuring channel). Similarly, light passing through the second optical coupling surface is totally reflected by the measuring portion M of the second side surface, reflected by the reflective portion RX of the first side surface back towards the second side surface, strikes the measuring portion M of the second side surface and is totally reflected to thereby form a second exit light beam for infrared spectroscopic measurement (second measuring channel). Each of the first and second side surfaces serves both as a measuring surface and as reflective surface for the infrared spectroscopic measurement. Thus it is possible to perform simultaneously two infrared spectroscopic measurements on two sides of the ATR prism. At least a portion of the remaining components of the infrared spectrometer can be used for both measuring channels. There may be, however, provided two infrared detectors for each measuring channel.

Further, Raman spectroscopy light is focused by each of the lenses 133 and 134 to an area of around a portion of the measuring surface close to the apex of the pyramid. In this example the portion of the measuring surface used for Raman spectroscopy measurement does not coincide with the measuring portion M used for infrared spectroscopic measurements. In other words, each of the optical measurements use different portion of the side surface. In the example shown in Fig. 23 and 24, the reflective portion RX is positioned between the measuring portion M for infrared spectroscopic measurement by means of double attenuated total reflection and the measuring portion for Raman spectroscopy.

In the example shown in Fig. 23 and 24, the ATR prism 51K has a two-sided pyramid form. Generally, the ATR prism may have more than two sides, for example be a 4-sided, 6-sided, etc. pyramid. Accordingly, there may be a corresponding number of measuring channels for simultaneously carrying out a plurality of spectroscopic measurements (n = 2, 4, etc.) by means of double attenuated total reflection. Generally, the ATR prism may have even number of sides and respective even number of measuring channels.

**Fig. 25** shows an example of an ATR prism 51L made from diamond having a four sided pyramid form with 4 channels for infrared spectroscopic measurement and additional 4 channels for Raman spectroscopy. **Fig. 26** shows a view from the bottom of the ATR prism 51L shown in Fig. 25 (i.e. from the side of the apex of the pyramid prism). The optical path of the infrared light for spectroscopic measurement is indicated as MIR. The optical path of the Raman spectroscopic light as RAS. The ATR prism 51L is otherwise similar to the one shown in Fig. 23.

**Fig. 27** shows an example of an ATR prism 51L having a six-sided pyramid form with 6 channels for infrared spectroscopic measurement and additional channels for Raman spectroscopy and optical coherence tomography. **Fig. 28** shows a cross-sectional view of the ATR prism 51 L shown in Fig. 27 along the line A-A'. The optical path of the infrared light for spectroscopic measurement is indicated as MIR-QCL-S, the optical path of the Raman spectroscopic light as RAS and the optical path of the optical coherence tomography (for example swept source spectral domain optical coherence tomography as SS-OCT-F).

In this example each optical coupling surface may be provided with two lenses: one for focusing Raman spectroscopy light and one for focusing optical coherence tomography light at respective areas of the side surfaces of the prism. In the example shown in Figs. 27 and 28, each side surface of the pyramid has a measuring portion/area for optical coherence tomography measurement, a measuring portion/area M for infrared spectroscopy by means of double attenuated total reflection and a measuring portion/area for Raman spectroscopy. Further each side surface comprises a reflective portion RX that may be arranged next to the measuring portion M for infrared spectroscopy. At least two of the measuring portions may at least partially overlap, for example the measuring portion/area for optical coherence tomography measurement may at least partially overlap with the measuring portion M for infrared spectroscopy by means of double attenuated total reflection. The arrangement of the measuring portions M and the reflective portions RX is such that at each measuring portion M the infrared light for spectroscopic measurement undergoes twice an attenuated total internal reflection.

An advantages of the multi-sided, multi-channel prisms shown above is that a 360° measurement can be made at each measuring site without having to turn the measuring probe at high angles.

The ATR prism may also be used as an optical coupling element for coupling light (for example intense laser light) for treatment of the measured tissue, if a malign tissue is detected. **Fig. 29** shows the beam path of the laser light emitted from a suitable light source that is used for treatment of a detected malign tissue 76 (for example a tumor). For a better understanding, only the beam path of the laser treatment part is shown. Similarly, one the elements of the ATR prism, measuring probe, etc. used for guiding the laser light to the treated site are shown. The system shown in Fig. 29 employs a four sided pyramid ATR prism 51L similar to the one shown in Figs. 25 and 26. In an example the laser light for the tumor treatment may be emitted from the same light source emitting Raman excitation light with the difference, that the intensity of the emitting light is increased about 10-fold. Further, the laser treatment light may use the same optical illumination path as the illumination path for the Raman spectroscopy (including the mirrors 131 and 132, the lenses 133 and 135 and parts of the side surfaces of the ATR prism 51L). Thus, tumor cells/tissue parts identified as being malign by the Raman spectroscopy (in combination with infrared spectroscopy by double attenuated total reflection) may be subjected to high energy laser light and destroyed immediately after their detection.

Of course it is also possible to use a dedicated laser light source and corresponding illumination path (that may comprise the mirrors 131 and 132, the lenses 133 and 135 and parts of the side surfaces of the ATR prism 51L and optionally other elements) to bring the laser light to the area to be treated. Advantageously, information about the exact position of the tumor cells/tissue parts obtained by the previous infrared spectroscopic measurements or combined infrared spectroscopic measurements may be used. As a light source an ultra-short pulse laser, for example a pico- or sub-pico pulse laser may be employed. The use of ultra-short pulse laser reduced the collateral damages in the neighboring (healthy) tissue.

In the above examples, the detected infrared spectroscopic data may be optionally combined with further morphological and spectral data from further optical measurements and may be processed to obtain one or more characteristic spectral features. Based on the obtained characteristic spectral features, it is possible to carry out a comparison with reference data of the characteristic spectral features of known objects. An unknown measured object may be then identified or classified in predetermined categories. For example tissue samples of different organs in different states (healthy, malign, etc.) examined previously by means of hystopathological analysis may be used to obtain reference data which may be used to classify the freshly cut tissue sample, for example during a surgery.

To be noted further is that in the middle infrared range, due to diffractive effects, the quality with which light beams may be collimated is lower than in the visible range. Thus, the light beam striking the ATR prism (incoming light beam) generally exhibits certain angular spread. However, the angular spread may be reduced considerably due to the high refractive index of the diamond, ZnSe, sillicone or germanium. This applies to all examples shown in the figures.

The computational aspects described here can be implemented in digital electronic circuitry, or in computer hardware, firmware, software, or in combinations of them. When appropriate, aspects of these systems and techniques can be implemented in a computer program product, for example tangibly embodied in a machine-readable storage device for execution by a programmable processor; and method steps can be performed by a programmable processor executing a program of instructions to perform functions by operating on input data and generating an output.

To provide for interaction with a user, a computer system can be used having a display device, such as a monitor or a LCD screen for displaying information to the user and a keyboard, a pointing device such as a mouse or a trackball, a touch-sensitive screen, or any other device by which the user may provide input to computer system. The computer system can be programmed to provide a graphical user interface through which the computer program(s) interact(s) with the user.

A number of embodiments have been described. Nevertheless, it will be understood that various modifications may be made. For example, the steps described can be performed in a different order and still achieve desirable results. Further, various elements disclosed in connection with a given example (such as for example the ATR prisms, the illumination and detection paths) may be combined with elements of other examples. Accordingly, other embodiments are within the scope of the claims.
Applicant: Universität Stuttgart
"A measuring probe, an apparatus and a method for label free attenuated reflection infrared spectroscopy"
Our Ref.: S12312EU - hb / br

**List of Reference Numerals**

| **Reference numeral** | |
|---|---|
| alpha_e1 | Angle of incidence of the light beam incident on the measuring portion of the measuring surface of the ATR prism prior to a reflection by the reflective portion of the reflective surface. The angle alpha_e1 is the angle between the main ray of the light beam incident on the measuring portion and the normal to the measuring portion. |
| alpha_e2 | Angle of incidence of the light beam incident on the measuring surface after a reflection by the reflective portion of the reflective surface. The angle alpha_e2 is the angle between the main ray of the reflected light beam incident on the measuring portion and the normal to the measuring portion. |
| alpha_p | Angle between the measuring portion of the measuring surface and the reflective portion of the mirror surface of the ATR-probe (prism angle). |
| alpha_av | Average value of the magnitudes of the two angles of incidence on the measuring portion of the measuring surface alpha_e1 and alpha_e2 (alpha_av = (alpha_e1 + alpha_e2)/2). |
| beta | Angle between the main ray of the light beam incident on the measuring surface of the ATR prism and the main ray of the exiting beam (i.e. the beam leaving the ATR prism after double total reflection at the measuring surface). The angle is twice the angle of incidence at the reflective portion of the reflective surface. |
| beta/2 | Angle of incidence at the reflective portion of the reflective surface of the ATR-prism |
| gamma | The angular beam spread due to dispersion |
| ATR | Attenuated Total Reflection / Area of attenuated total reflection |
| Non-ATR | Non-Attenuated Total Reflection /Area of non-attenuated total reflection |
| OV | Overlapping area of attenuated and non-attenuated total reflection |
| CLT | Center of the beam waist |
| LA | Longitudinal axis of the probe |
| M | Measuring portion of the measuring surface of the ATR prism |
| RX | Reflective portion of the reflective surface (endreflector surface) of the ATR prism |
| KA1 | Optical coupling surface of the ATR prism |
| KA2 | Optical coupling surface of the ATR prism |
| KA3 | Optical coupling surface of the ATR prism |
| KA4 | Optical coupling surface of the ATR prism |
| MIC | Microscopic optical path |
| MIR-QCL-S | MIR Quantum cascade laser beam path (preferably, the beam is weakly focused) |
| RC | Optical channel for Raman spectroscopy |
| R-F | Raman spectroscopic light exhibiting strong focusing |
| SS-OCT-F | Swept-Source OCT bean path exhibiting moderate focusing |
| SBE | Optical coupling surface of the ATR prism for the infrared spectroscopy |
| SE | Light beam for the ATR-measurement |
| F_RA | Focus of the Raman excitation light |
| F_OCT | Focus of the OCT measuring light |
| F_Cam | Focal plane of the camera ray path |
| MIR | Beam path of the infrared light (for example in the mid-infrared) |
| MS | Micro-vibrations of the ATR prism |
| oB | Upper part of the optical arrangement shown in Figs. 17 and 18 |
| OCT-S | OCT beam path (OCT measurement path) |
| RAS | Raman spectroscopy beam path (Raman spectroscopy measurement path) |
| RSL | Raman scattered light |
| uB | Lower part of the arrangement shown in Figs. 17 and 18 |
| sigma | Wavenumber [cm⁻¹] |
| TR | Total reflection |
| 2w₀ | Beam waist diameter |
| 1 | Quantum cascade laser battery, preferably in infrared |
| 2 | Beam splitter |
| 3 | Beam forming optical system (preferably forms the beam waist in the ATR prism, preferably at the reflective portion RX) |
| 11 | Infrared beamline of a synchrotron |
| 12 | Broadband light source emitting , monochromatic or quasi-monochromatic light with a variable (scannable) wavelength, for example quantum cascade laser battery (optionally with a collimator output) |
| 15 | Laser (for example quantum cascade laser) |
| 20 | Broadband light source emitting light with continuous or quasi-continuous spectrum (for example a quantum cascade laser battery, optionally exhibiting an infrared fiber output). |
| 21 | Monochromator (for example fiber coupled), preferably in the mid-infrared range from about 900 cm⁻¹ to 1800 cm⁻¹ |
| 22 | Fiber based optical coupling system with a collimator output for beam expansion |
| 23 | Fiber based optical coupling system with a collimator output for beam expansion |
| 23A | Fiber based optical coupling system |
| 24 | Silver-halide fiber |
| 25 | Hyper-spectral modulator |
| 26 | Elastic infrared optical fiber |
| 27 | Optical system for adjustment of the beam's cross section |
| 29 | Stainless steel sheathing/housing |
| 30 | Infrared detector (for example a spatially and spectrally detecting MIR detector) |
| 30A | Detector system comprising an achromatic optical focusing system, a fiber and a detector |
| 31 | Reference detector (for example a mercury-cadmium telluride (MCT) detector). |
| 32 | Optical system of the reference detector |
| 33 | Optical system of the detector (e.g. fiber based) |
| 34 | Optical system of the detector |
| 34A | Detector assembly |
| 35 | Collimator lens |
| 36 | Focusing lens |
| 40A | Coupling lens |
| 40B | Lens prism with a blazed diffractive grating |
| 42 | Blazed diffractive grating |
| 43 | Elongated optical member with a curved (lens) surface 44 (preferably with optically separated light entry and light exit) |
| 44 | Curved (lens) surface |
| 45 | Elongated optical member |
| 46, 46A, 46B | One-sided reflective coating (e.g. aluminum reflective coating) |
| 47A, B | One-sided reflective coating (e.g. aluminum reflective coating) |
| 48 | Measuring surface having a reflecting portion (by means of non-attenuated total reflection) |
| 50, 50A, 50B, 50B, 50C, 50D, 50E | ATR prism (preferably with an optical function and a cutting function and preferably made of diamond) |
| 51A, 51B, 51C, 51D, 51E | ATR prism (preferably with an optical function and a cutting function and preferably made of diamond) |
| 51G, 51H | ATR prism (preferably with an optical function and a cutting function and preferably made of silicon) |
| 51K, 51L, 51M | ATR prism having multiple measuring and reflective surfaces (e.g. 2-channel. 4-channel, 6-channel, etc. ATR prism) |
| 52 | Reflective layer (may have a multilayer structure, preferably with hard coating outermost layer) |
| 52A | Reflective layer (may have a multilayer structure, preferably with hard coating outermost layer) |
| 52B | Reflective layer (may have a multilayer structure, preferably with hard coating outermost layer) |
| 53 | Cutting blade of the ATR prism |
| 54 | Cutting blade of the ATR prism |
| 55 | Optical component for adjusting the cross sectional of the incident optical beam (for example mirror-based). |
| 56 | Optical component for adjusting the cross sectional of the output optical beam (for example mirror-based) |
| 57 | First off-axis parabolic mirror |
| 58 | Second off-axis parabolic mirror |
| 59 | Mirror |
| 60 | Computer controlled drive system (e.g. a computer controlled translational or linear stage) |
| 61 | Computer controlled (balanced) drive system (e.g. a computer controlled rotational stage) |
| 63 | Beam splitter plate (e.g. from ZnSe) |
| 64 | Beam splitter layer (e.g. adapted for the MIR spectral range) |
| 65 | Rotary stage for alignment of the optical system |
| 66 | Triple-mirror type reflector (e.g. hollow cube reflector) |
| 67 | Roof-edge type mirror |
| 68A | Translational mirror based Interferometer employing triple mirrors |
| 68B | Rotating mirror based Interferometer with triple reflectors |
| 69 | A single-side gold reflective coating |
| 70 | Blazed diffractive grating |
| 71 | Uncut human soft tissue |
| 72 | Cut human soft tissue in close contact with the measuring portion |
| 73 | Stomach tissue undergoing surgical operation |
| 74 | Surface of the organ to be examined |
| 75 | Skin surface |
| 76 | Tumor subjected to a laser treatment |
| 78 | Small tumor |
| 79 | Human tissue subjected to surgical treatment |
| 82 | Beam splitter |
| 83 | Mirror |
| 91 | Raman excitation laser |
| 92A | Monomode fiber in the illumination path |
| 92B | Monomode fiber outside of the measuring probe, arranged downstream of the Y fiber coupler |
| 92C | Monomode fiber within the ATR probe |
| 92D | Monomode fiber in the detection path |
| 93 | Y fiber coupler |
| 94 | Notch-Filter |
| 95 | Optical transfer system |
| 96 | Fourier spectrometer for a scattered Raman light |
| 97 | Dispersive one-channel spectrometer for scattered Raman light |
| 98 | Optical imaging system for illumination and image forming in a camera |
| 99 | Off-axis ellipsoid mirror |
| 101 | Source and Fourier spectrometer assembly |
| 102 | Fiber based optical coupling for a plurality of measuring probes |
| 103 | Kit of measuring probes |
| 104 | Detector assembly |
| 111 | Mirror |
| 112 | Collimating lens |
| 115 | Illumination/Camera assembly |
| 119 | Optical coupling system |
| 120 | Monomode fiber |
| 131 | Mirror |
| 132 | Mirror |
| 133 | Focusing lens having a diffractive element |
| 135 | Focusing lens having a diffractive element |
| 141 | Beam splitting system for a plurality of measuring channels |
| 142 | Array of measuring probes |
| 143 | Measuring probe |
| 143a, 143b, 143c, ... | Measuring probes |
| 145 | Computerized mechanical driving system |
| 146 | Control system (including for example a control system) |
| 147 | Display |
| 148 | Data googles or spectacles or Head-up Display/Head-Mounted-Display |

## Claims

1. A measuring probe (143; 143a-c) for infrared spectroscopy, said measuring probe (143, 143a-c) having an elongated form with a first end for coupling and decoupling infrared light into and out of the measuring probe (143; 143a-c) and a second end, wherein said measuring probe (143; 143a-c) comprises:
an ATR prism (50, 50A-E, 51A-M) arranged at the second end of the measuring probe (143; 143a-c), said ATR prism (50, 50A-E, 51A-M) having
at least one first surface having at least one measuring portion (M) configured to be brought in optical contact with a measured object (72),
at least one second surface having at least one reflective portion (RX), and
a cutting portion (53; 54) for cutting through the measured object (72), said cutting portion having a cutting tip or cutting blade with a cutting angle of equal to or less than 60°,
said ATR prism (50, 50A-E, 51A-M) being configured so that
at least a portion of the infrared light entering the measuring probe (143; 143a-c) undergoes an attenuated total reflection at the least one measuring portion (M) of the first surface,
at least a portion of the totally reflected light is reflected back towards the first surface by the at least one reflective portion (RX) of the second surface, and
at least a portion of the light reflected back by the at least one reflective portion (RX) of the second surface undergoes an attenuated total reflection at the at least one measuring portion (M) of the first surface and is decoupled from the ATR prism (50, 50A-E, 51A-M).

2. A measuring probe (143, 143a-c) according to claim 1, wherein the angle between the measuring surface and the longitudinal axis (LA) of the measuring probe (143, 143a-c) is equal to or less than 60°.

3. A measuring probe (143, 143a-c) according to any one of the preceding claims,
wherein the cutting portion (53; 54) is formed by an intersection of the first surface and the second surface of the ATR prism (50, 50A-E, 51A-M) or wherein the cutting portion (53; 54) is formed as an additional element; and/or
wherein the cutting angle is preferably equal or less than 45°, preferably equal or less than 25°.

4. A measuring probe (143, 143a-c) according to any one of the preceding claims, wherein the difference between the magnitude of the angle alpha_p between the first surface and the second surface of the ATR prism (50, 50A-E, 51A-M) and the magnitude of the critical total reflection angle is equal to or less than 12°.

5. A measuring probe (143, 143a-c) according to any one of the preceding claims, wherein the ATR prism (50, 50A-E, 51A-M) is made of diamond, crystalline silicon or crystalline germanium.

6. A measuring probe (143, 143a-c) according to any one of the preceding claims, wherein the first and/or the second surface comprises at least one reflective portion (RX), configured to reflect incident light by means of non-attenuated total reflection towards the other surface of the ATR prism (51 G; 51 H).

7. A measuring probe (143, 143a-c) according to any one of the preceding claims, wherein the first surface comprises a plurality of measuring portions (M).

8. A measuring probe (143, 143a-c) according to any one of the preceding claims, wherein the second surface comprises a transparent portion.

9. A measuring probe (143, 143a-c) according to any one of the preceding claims, wherein the ATR prism (51 K; 51 L; 51 M) further comprises a plurality of first surfaces, each having at least one measuring portion (M) configured to be brought in optical contact with a measured object (72).

10. A measuring probe (143, 143a-c) according to any one of the preceding claims, wherein the ATR prism (50; 50B; 50C; 50D; 50E; 51K; 51 L; 51 M) comprises:
an optical coupling surface (SBE) for coupling infrared spectroscopic light into the ATR prism (50; 50B; 50C; 50D; 50E; 51 K; 51 L; 51 M); and
at least one optical coupling surface for coupling light for a further optical measurement into the ATR prism (50; 50B; 50C; 50D; 50E; 51K; 51L; 51 M), said light for further optical measurement including one or more of Raman excitation light, light for optical coherence tomography measurements, swept-source spectral-domain optical coherence tomography measurements and light for microscopic observations.

11. An apparatus for infrared spectroscopy, comprising
a light source (11; 12; 20),
a detector (30),
a measuring probe (143, 143a-c) according to any one of the preceding claims.

12. An apparatus for infrared spectroscopy according to claim 11, further comprising a plurality of measuring probes (143a, 143b, 143c), each measuring probe (143a, 143b, 143c) comprises an ATR prism (50, 50A-E, 51A-M), wherein the ATR prisms of the different probes have the same or different angles between the measuring and the reflective surface.

13. A method for infrared spectroscopy by using an apparatus for infrared spectroscopy according to any one of claims 11 or 12, said method comprising:
bringing the at least one measuring portion (M) of the first surface of the ATR prism (50, 50A-E, 51A-M) in optical contact with the measured object (72);
illuminating the at least one measuring portion (M) with incident infrared light, so that at least a portion of the incident light undergoes an attenuated total reflection at the at least one measuring portion (M),
reflecting, by the at least one reflective portion (RX) of the second surface, at least a portion of the totally reflected light back towards the measuring portion (M), whereby at least a portion of the light reflected back by the reflective portion (RX) undergoes an attenuated total reflection by the at least one measuring portion (M) of the first surface,
decoupling the totally reflected light from the ATR prism (50, 50A-E, 51A-M),
detecting at least a portion of the light exiting the ATR prism (50, 50A-E, 51A-M).

14. A method for infrared spectroscopy according to claim 13, further comprising moving the measuring probe (143; 143a-c) to a new region of the measured object (72) and repeating the steps of bringing the at least bringing the at least one measuring portion (M) in optical contact with the measured object (72), illuminating the at least one measuring portion (M), reflecting, by the at least one reflective portion (RX) of the reflective surface at least a portion of the totally reflected light back towards the measuring portion (M), decoupling the totally reflected light from the ATR prism (50, 50A-E, 51 A-M), and detecting at least a portion of the outgoing light.

15. A method for infrared spectroscopy according to claim 13 or 14, further comprising:
illuminating at least one portion of the first surface of the ATR prism (50; 50B; 50C; 50D; 50E; 51 K; 51 L; 51 M) that is in contact with the measured object (72) with light for an additional optical measurement, wherein the light for the additional optical measurement is incident perpendicularly on the illuminated portion or at an angle deviating from the perpendicular incidence by no more than ± 15°, preferably by no more than ± 12°, and detecting at least a portion of the light for additional optical measurement returned from the measured object (72), wherein said additional optical measurement includes one or more of Raman spectroscopy, optical coherence tomography, swept-source spectral-domain optical coherence tomography measurements and microscopy; and/or
illuminating the at least one portion (M) of the first surface of the ATR prism (50; 50B; 50C; 50D; 50E; 51 K; 51 L; 51 M) that is in contact with the measured object (72) with additional light for a treatment of the measured object (72), wherein the light for a treatment of the measured object is incident perpendicularly on the illuminated portion or at an angle deviating from the perpendicular incidence by no more than ±15°, preferably by no more than ±12°.
